(19) Europäisches Patentamt
European Patent Office
Office européen des brevets

(11) Publication number: 0 390 530 B1

# (12) EUROPEAN PATENT SPECIFICATION

(45) Date of publication of patent specification: 17.05.95

(51) Int. Cl.[6]: C12P 21/08, C07K 14/435, C07K 16/30, C12N 5/18, G01N 33/577

(21) Application number: 90303297.7

(22) Date of filing: 28.03.90

## (54) Retinoblastoma gene product antibodies and uses therefor.

(30) Priority: 31.03.89 US 332082

(43) Date of publication of application:
03.10.90 Bulletin 90/40

(45) Publication of the grant of the patent:
17.05.95 Bulletin 95/20

(84) Designated Contracting States:
AT BE CH DE DK ES FR GB GR IT LI LU NL SE

(56) References cited:
WO-A-89/06703

BIOLOGICAL ABSTRACTS, vol. 86, no. 8, 1988, Biological Abstracts Inc., Philadelphia, PA (US); J.A. DECAPRIO et al., no. 82383&NUM;

BIOLOGICAL ABSTRACTS, vol. 84, no. 4, 1987, Biological Abstracts Inc., Philadelphia, PA (US); T. MATSUMURA et al., no. 37550&NUM;

(73) Proprietor: CANJI, Inc.
3030 Science Park Road
Suite 302
San Diego
CA 92121-0177 (US)

(72) Inventor: Fung, Yuen Kai
2455 Ivanhoe
Los Angeles, CA 90030 (US)
Inventor: T'Ang, Anne
2455 Ivanhoe
Los Angeles, CA 90030 (US)

(74) Representative: Wilkinson, Stephen John et al
Stevens, Hewlett & Perkins
1 St. Augustine's Place
Bristol BS1 4UD (GB)

EP 0 390 530 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

FIELD OF THE INVENTION

This invention relates to the field of tumor cell detection, antibodies to the protein coded by the retinoblastoma gene (RB1), methods for detecting the RB1 protein in tumors, methods of diagnosing tumor progression in a variety of tumor types and cell types which are deficient in the retinoblastoma gene.

BACKGROUND ON THE INVENTION

Tumor development in certain types of cancer is thought to be the result of the unmasking of one or more recessive 'tumor supppressor' genes by mutation. Two childhood cancers, retinoblastoma and Wilm's tumor, are the prototypes for this type of cancer. Retinoblastoma is a rare ocular malignancy, occurring at a frequency of approximately one in 20,000 live births. Retinoblastoma occurs as both an inherited and a sporadic disease, with around 40% of cases being hereditary. Cytogenetic studies identified the chromosomal region involved in retinoblastoma as 13q14. Recently the retinoblastoma gene (RB1) has been cloned and sequenced. In at least 40% of primary retinoblastomas, there is a clear structural abnormality to RB1, the types of abnormality including deletions of the entire gene, smaller internal deletions, translocations and point mutations.

Clinically, patients surviving retinoblastoma are at a significantly increased risk of developing other specific types of primary tumors, predominantly osteosarcomas, but less frequently fibrosarcomas and possibly melanomas. In addition, unaffected family members develop other cancers more frequently than expected, and these cancers can be of a variety of types. Analysis of DNA from second primaries from retinoblastoma patients shows clear abnormalities in both alleles of the RB1 gene. Furthermore, DNA isolated from fibrosarcomas and osteosarcomas from patients with no history of retinoblastoma also shows deletion of the RB1 gene. Thus, loss of function of the RB1 gene may have a general role in human oncogenesis.

Loss of function of the RB1 gene has also been associated with retinoblastomas, osteosarcomas, fibrosarcomas, carcinomas of breast, ovary, bladder, lung, cervix and in soft tissue sarcomas. Structural abnormalities have also been detected in 13% of primary small cell lung carcinomas (SCLC), and in 18% of SCLC cell lines. More significantly, loss of RB1 mRNA was seen in 60% of the SCLC lines. SCLC, like retinoblastoma, is a tumor of neuroendocrine origin. Frequent aberrations to the RB1 gene have also been detected in primary carcinoma of the breast. The involvement of the RB1 gene in two tumor types displaying phenotypic properties of neuroendocrine differentiation may be of significance. Breast carcinomas are not, however, of neuroendocrine origin, but they can show a strong familial trend, with some families showing a very strong genetic component. While there is evidence that mothers of children with osteosarcoma and soft issue sarcoma show a higher rate of breast cancer, there is no direct evidence of any association between breast cancer and retinoblastoma.

The structure of the RB1 gene product (the RB1 protein) has been deduced from sequence analysis of RB1 cDNA. The longest RB1 reading frame encodes a protein of 928 amino acids. Using antisera against a tryp E-RB fusion protein, a protein of an apparent molecular weight of 110,000 to 114,000 daltons was immunoprecipitated. The RB fusion protein is a phosphoprotein. Antisera prepared against either synthetic oligopeptides or bacterially expressed peptides immunoprecipitate a protein of approximately 105 kDalton which is believed to be the native form of Rb1 protein.

The cDNA sequence coding for the RB1 protein (shown on Figure 1) reveals the presence of a stretch of amino acids in the protein at position 609 to 615 (VRSPKKK), which is reminiscent of the nuclear translocation signal found in SV40 large T antigen and in Polyoma large T antigen. This implies that the RB protein is a nuclear protein. The RB1 protein has been detected by immuno-staining and by sub-cellular localization by fractionation of cellular components in the nucleus and in the nuclear fraction, respectively.

The detection of alterations in the RB1 gene discussed above have been obtained utilizing DNA probes and DNA hybridization assays. Such techniques are generally known to those of skill in the art.

Biological Abstracts, Vol 86, No 8, 1988, Abstract No 82383 and Cell 54(2): 275-283, 1988 discloses the formation of a specific complex between SV40 large tumor antigen and the product of the retinoblastoma susceptibility gene.

WO-A-8906703, which was published on 27 July 1989, i.e., after the priority date of this application, teaches the retinoblastoma polypeptide and methods for detecting a defective retinoblastoma gene in human patients.

Utilization of DNA hybridization techniques for detecting gene aberrations is limited in its sensitivity since the presence of individual cells deficient in the RB1 gene can not be detected in the DNA preparations utilized to carry out the DNA hybridization techniques. In addition, DNA hybridisation assays are time consuming and thus, detection of the presence or absence of the RB1 gene by DNA hybridization techniques are further hindered by the length of time necessary to obtain results.

Until now a sensitive, specific and rapid method for detecting RB1 gene aberrations in tumor tissue specimens has not been available. Such a method is desirable in order to aid in ascertaining the presence or absence of a functional RB1 gene and thereby assess the prognosis of Retinoblastoma tumor patients, as well as in deciding on a treatment regimen.

## SUMMARY OF THE INVENTION

An object of the present invention is to provide a sensitive method for detecting tumors which have an altered retinoblastoma gene. Another object of the present invention is to provide a polyclonal antibody for detecting the protein product of the retinoblastoma gene (RB1 protein). It is a further object of the present invention to provide a monoclonal antibody for the detection of the RB1 protein. An additional object of the present invention is to provide a hybridoma which produces monoclonal antibody to the RB1 protein.

It is a further object of the present invention to provide an additional method for determining the grade and stage of a variety of tumors selected from the group consisting of retinoblastomas, osteosarcomas, fibrosarcomas, carcinomas of breast, ovary, bladder, lung, cervix and in soft tissue sarcomas. All of these tumors have shown subpopulations of cells which have been associated with altered or defective retinoblastoma gene.

It is a further object of the present invention to provide an additional criterion for determining the grade and stage of a variety of tumors and, thereby aid in assessing the prognosis and future treatment regimen for the tumor patient.

Thus in accomplishing the foregoing objects, there is provided in accordance with one aspect of the present invention an antibody having specific binding affinity to the RB1 protein product of the retinoblastoma gene produced by immunising a host animal with a peptide located at or near the c-terminus of the RB1 protein sequence.
Preferably the sequence of said peptide is selected from:

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$- Cys-Glu-Glu-Ile-Tyr-Leu-Lys-Asp-Lys-Asp-Leu-Asp-Ala-Arg-Leu-Phe-Leu-Asp-His-Asp-Lys,

and

$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys.

The invention also provides a murine hybridoma which produces a monoclonal antibody specifically immunoreactive with the RB1 protein and having specific binding affinity to a peptide sequence selected from:

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$- Cys-Glu-Glu-Ile-Tyr-Leu-Lys-Asp-Lys-Asp-Leu-Asp-Ala-Arg-Leu-Phe-Leu-Asp-His-Asp-Lys,

and

$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys.

In a further embodiment, the invention relates to monoclonal antibody RB1-MAB P2 producible by the hybridoma of the invention. The hybridoma is deposited as ATCC HB 10093.

In another embodiment, a method of detecting the RB1 protein is provided comprising the steps of preparing tissue sections from tumor tissue, incubating the antibodies (either monoclonal or polyclonal) with said prepared tissue sections to form an antibody-antigen complex and detecting the formation of the antigen-antibody complex so formed.

Another embodiment of the invention provides a method for detecting subpopulations of cells which are associated with tumors such as osteosarcomas, fibrosarcomas, melanomas, carcinoma of the breast, ovary, bladder, lung, cervix and soft tissue sarcomas and a method for diagnosing and determining the prognosis in individuals containing such tumors. The method involves biopsying the tumor, preparing tissue sections from the biopsy tumor tissue, incubating the prepared tissue sections with RB1 monoclonal or polyclonal antibody of the present invention to allow the antibody to specifically bind to the tissue and the formation of an antibody-antigen complex and detecting the formation of antibody-antigen complex.

The present invention is useful in detecting a wide variety of tumors which have a defect in the retinoblastoma gene product (RB1 protein) and for the assessment of the prognosis and future treatment regimen of the tumor patient.

Other and further objects, features and advantages will be apparent from the following description of the presently preferred embodiments of the invention, given for the purposes of disclosure when taken in conjunction with the accompanying drawings.

## BRIEF DESCRIPTION OF THE DRAWINGS

The invention will be more readily understood from a reading of the following specification and by reference to the accompanying drawings forming a part thereof:

Figure 1 shows the RB1 cDNA sequences including part of the genomic sequence immediately 5' of the cDNA. The A residue in the ATG codon is defined as position 1. The amino acids of the longest reading frame are shown below each of the coding sequences.

Figure 2 demonstrates the precipitation of labeled RB1 protein by polyclonal antiserum RB-AB20 and the IgG fraction purified from RB-AB20.

Figure 3 demonstrates the immunohistochemical staining of an RB1 positive cell line, (SW613) and an RB1 negative cell line (MDA-MB468) with monoclonal antibody RB-MAB20.

Figure 4 shows the immunohistochemical detection of RB1 protein in human breast tissue. All magnifications X250.

The drawings are not necessarily to scale and certain features of the invention may be exagerated in scale or shown in schmatic form in the interest of clarity and conciseness.

## DETAILED DESCRIPTION

Antigens are compounds capable of eliciting an immune response and the concommittant production of antibodies which react with and bind to the target antigen with some specificity. Antibody probes specific for antigens such as viruses or specific determinants thereof, peptides and proteins derived from a variety of sources, carbohydrate moieties and a wide variety of biopolymers are known to those of skill in the art. The general methods for preparation of such antibodies are also known to those of skill in the art.

Briefly, polyclonal antibodies may be prepared by immunization of an animal host with a peptide sequence coded by the RB1 gene. Preferably, the antigen is a peptide sequence located at or near the hydrophilic c-terminus of the RB1 protein. The antigen is administered to the host subcutaneously at weekly intervals followed by a booster dose one month after the final weekly dose. Subsequently, the serum is harvested from the host and stored until further use. The RB1 polyclonal antiserum may be use directly in the immunohistochemical procedures of the present invention. Alternatively, RB1 antibodies may be precipitated from the serum by means known to those skilled in the art and used in a more concentrated form or alternatively, the RB1 antibodies may be purified by affinity technique and used in this purified form. The antibodies may also be detectably labeled by techniques known to those of skill in the art and further discussed below.

Fusion between myeloma cells and spleen cells from immunized donors has been shown to be a successful method of deriving homogeneous antibodies. Thus, continuous cell lines of genetically stable hybridoma cells capable of producing large amounts of monoclonal antibodies against various antigens have been developed. According to U.S. Patent No, 4,172,124 to Koprowski et al., antibodies demonstrating a specificity for malignant tumors can be produced by somatic cell hybrids between hypoxanthine phosphoribosyltransferase deficient myeloma cells and spleen or lymph cells derived from an animal previously primed with tumor cells. Also, according to U.S. Pat. No, 4,196,265 to Koprowski et al., continuous cell lines of genetically stable fused cell hybrids capable of producing large amounts of monoclonal antibodies against specific viruses and their antigenic determinants have been developed.

Such cell fusion techniques can also be employed to provide a reliable and standard supply of anti-RB1 antibodies. According to the present invention novel continuous hybridoma cell lines which expresses anti-RB1 antibody is obtained by immunizing an animal with a peptide sequence corresponding to an amino acid sequence at or near the hydrophilic c-terminus of the RB1 gene product, preferably to a synthetic sequence selected from the group consisting of the P2, P3, P4 or P5 RB1 peptides shown on Figure 1, forming fused hybrid cells between antibody-producing cells from the immunized animal and myeloma cells, cloning the hybrids and selecting clones wich express anti-RB1 antibody. More specifically, a mouse or other animal is injected with purified RB1 antigen and the antibody producing cells of the animal's spleen are then fused with a cancerous type of mouse cell or myeloma cell. The hybrid cell so formed produces the anti-RB1 antibody molecule of its spleen cell parent and continually grows and divides like its parent myeloma cell. The clone of cells producing such antibody are selected and grown as a continuous cell line from which large amounts of anti-RB1 antibody is harvested. One such clone designated RB1-MABP$_2$ is further discussed below.

In the alternative, the clonal hybrid cells may be injected into a histocompatable animal where they proliferate, producing high levels of anti-RB1 antibody which can be recovered from the animal's ascites fluid.

Thus, the present invention makes available on a relatively large scale a reliable and standard supply of anti-RB1 antibody for use in immunohistochemical detection of RB1 protein expression, or, in the alternative, the absence of RB1 protein expression in a tumor sample where the absence of the protein is associated with a particular type, grade or stage of tumor. The monoclonal antibodies so prepared may also be labeled by procedures known to those of skill in the art.

Detectable labels may be any material having a detectable physical or chemical property. Such detectable labels have been well-developed is the field of immunoassays and in general almost any label useful is such methods can be applied to the present invention. Particularly useful are enzymatically active groups, such as enzymes (such as, for instance, alkaline phosphatase and peroxidases)(see Clin. Chem., 22:1243 (1976)), enzyme substrates (see British Pat. Spec. 1,548,741), coenzymes (see U.S. Patent Nos. 4,230,797 and 4,238,565) and enzyme inhibitors (see U.S. Patent No. 4,134,792); fluorescers (see Clin. Chem., 25:353 (1979)); chromophores; luminescers such as chemiluminescers and bioluminescers (see Clin. Chem., 25:512 (1979)); and radioisotopes. Commonly used detectable radioactive labels include, but not limited to, $^{32}P$, $^{14}C$, $^{125}I$, $^{3}H$ and $^{35}S$. The biotinylated probes are detected after hybridization using avidin/streptavidin, fluorescent, enzymatic or collodial gold conjugates. Antibodies may also be labeled with other fluorescent compounds, with immunodetectable fluorescent derivatives or with biotin analogues. Indirect fluorescent immunocytochemical procedures may also be utilized.

In order to detect the formation of antigen-antibody complexes, a detectable label is utilized. The detectable label (or reporter molecule) may be directly attached to the anti-RB1 antibody molecule prior to incubation with the target tissue. Alternatively, unlabeled RB1 antibody may be incubated with the target tissue and the antigen-antibody complex detected by the addition of a second, detectably labeled, antibody which binds to the RB1 antibody complexed with the RB1 tissue antigen.

The presence or absence of the RB1 protein in specific cells of a tumor tissue sample may be determined directly by utilizing a reporter molecule which can be visually detected, or indirectly by utilizing a reporter molecule such as a radioactive label and superimposing the developed autoradiograph on a visual representation of the tissue section. One can then count the RB1 positive and negative tumor cells to determine the number of tumor cells expressing the RB1 protein.

One embodiment of the present invention utilizes a polyclonal antibody that binds to the RB1 protein. Polyclonal antibodies to the RB1 protein (RB1-AB20) were prepared by immunizing rabbits with peptide sequences corresponding to a hydrophilic part of the RB1 protein located at or near the c-terminus of the protein sequence deduced from the human RB1 cDNA sequences as shown on Figure 1. In a preferred embodiment, a synthetic peptide (hereinafter termed "P5") corresponding to 23 amino acid residues was prepared.

Three other peptide sequences were also synthesized. The amino acid sequences of the synthetic RB1 peptides are designated as follows:

P2: $HN_2$- Cys-Arg-Met-Gln-Lys-Gln-Lys-Met-Asn-Asp-Ser-Met-Asp-Thr-Ser-Asn-Lys-Glu-Glu-Lys.

P3: $HN_2$- Cys-Arg-Thr-Pro-Arg-Arg-Gly-Gln-Asp-Arg-Ser-Ala-Arg-Ile-Ala-Lys-Gln-Leu-Glu-Asp-Asp,

P4: $HN_2$- Cys-Glu-Glu-Ile-Tyr-Leu-Lys-Asp-Lys-Asp-leu-Asp-Ala-Arg-Leu-Phe-Leu-Asp-His-Asp-Lys, and

P5: $HN_2$- Cys-Lys-Pro-Leu-Lys-Lys-Leu-Phe-Arg-Asp-Ile-Glu-Gly-Ser-Asp-Glu-Ala-Asp-Gly-Ser-Lys.

A cysteine residue was added to the N-terminal of each of the peptides for coupling purposes. Thus N-terminal cysteine moiety is not party of the RB1 protein sequence.

The polyclonal and monoclonal antibodies can be used to detect the presence of, or absence or alteration of the RB1 protein. In one embodiment, a method for detecting the RB1 protein comprises the steps of preparing tissue sections from tumor tissue biopsies, incubating the monoclonal or polyclonal antibodies with the prepared tissue sections to form an antibody-antigen complexes and then detecting the formation of the antigen-antibody complex.

In another embodiment of the present invention subpopulations of cells deficient in the RB1 protein can be identified in tumor tissues utilizing the immunohistochemical described above.

Since tumor cells in the advanced stages of disease show more retinoblastoma protein defects, the method can be used for the diagnosis of the stage of the tumor cells as well as the prognosis for the individual containing said tumors.

Current treatment decisions for individuals are frequently based on specific prognostic parameters. The major prognostic factors for breast cancer, for instance, include the presence or absence of tumor in axillary nodes, and the presence or absence of hormonal receptors in the tumor cell. Other prognostic factors often associated with disease relapse and overall patient survival include the size of the primary lesion, and stage of the disease at diagnosis. Slamon has postulated that HER-2/neu gene amplification had greater prognostic value as a predictor of both over all survival time and time to relapse in breast cancer patients than any other conventionally use criterion except lymph node involvement. (Science 235:177(1987)

The method of the present invention can be used to assess the stage and grade of a wide variety of tumors. For example, the defect has been found in retinoblastomas, osteosarcomas, fibrosarcomas, carcinomas of breast, ovary, bladder, lung, cervix and in soft tissue sarcomas. All of these tumors have defects of the RB gene at the DNA level and some have shown subpopulations of cells which have been associated with altered or defective retinoblastoma gene. Thus, the absence of detectable RB1 protein can be utilized as one identifying criteria in these tumors. Since the degree of loss of the RB1 protein expression increases with increase in the grade of tumor as identified by other conventional methods in breast tumors, the present invention provides a further criterion for determining the grade and stage of such tumor. This method will provide an additional criterion for determining the prognosis and future treatment course for the tumor patient.

Having now generally described the invention, a more complete understanding can be obtained by reference to the following specific examples. These examples are provided for purposes of illustration only and are not intended to be limiting unless otherwise specified.

Example 1

RB1 Antibody Production

Polyclonal antibodies were prepared by immunising individual New Zealand female rabbits with one of the RB1 peptides, $P_2$, $P_3$, $P_4$ or $P_5$. The rabbits were boosted at 2 week intervals and the serum collected was used as a source of the antibodies. One antiserum RB1-AB20, raised against the peptide $P_5$ was used for subsequent immunostaining procedures as described in Example 2. The RB1-AB20 antibody is of the IgG subtype. The RB1-AB20 antiserum precipitated RB1 protein from $^{32}P$ labelled cells as shown on Figure 2, lane 1. The IgG fraction of the polyclonal antiserum, purified by conventional techniques, also precipated the RB1 protein as shown on Figure 2, lane 2.

The hybridomas of the present invention were prepared as follows. Balb/c mice were injected directly into the spleen with 200mg of the subject peptide dissolved in PBS. The immunized animals were sacrificed three days later and the spleen cells were harvested. Spleen cells ($10^8$) were mixed for 2 min. with the mouse myeloma NS-1 cells ($10^7$) in 0.8 ml 50% polyethylene glycol (PEG 4000). After sitting undisturbed for 1 min., the cells were resuspended in 70 ml HAT (Hypoxanthine-aminopterin-thymidine) medium containing 20% fetal calf serum. The cells were then plated in 96 well culture dishes and incubated at 37 °C in a humidified $CO_2$ atmosphere. Positive hybridoma colonies were screened by ELISA technique using the immunizing antigens to detect RB1 antibody producing hybridoma cells. Elisa positive colonies were plated at serial dilutions and the Elisa test repeated. After several cycles, positive hybridoma cell lines were injected intraperitoneally into Balb/c mice and grown as ascites fluid. The ascites fluid was used as a source of the hybridoma. Several cell lines, including one designated RB1-MAbP2, were found to produce RB1 antibodies and to be useful for immunohistochemical staining of tumor tissues. Hybridoma cell lines were preserved in liquid nitrogen.

The hybridoma cell line designated RB1-MAbP2 was deposited with the American Type Culture Collection (ATCC), Rockville, Md., U.S.A., Deposit Accession No. HB10093 on March 31, 1989. The deposits are available pursuant to the patent laws and regulations of the United States and of those countries foreign to the United States in which counterparts of this application are filed. The availability of a deposit does not constitute a license to practice the invention of this application in derogation of any patent issued thereon or on any division or continuation of this application.

Example 2

Immunohistochemical localization of the RB1 Protein

The correlation of the presence and absence of RB1 protein with the presence or absence of the RB1 gene was demonstrated utilizing the RB1 antibodies of the present invention. Tissue culture cells, including cell lines SW613, a RB1 gene positive breast tumor cell line, and MDA-MB468, a RB1 gene negative breast tumor cell line, were cultured for 48 hrs. in RPMI 1640 medium supplemented with 20% fetal calf serum and 0.1% Pennicillin/Streptomycin solution. The cells were rinsed twice with cold PBS. The cells were fixed with 5% acetic acid in 95% ethanol for 6 min. at 4 °C.

After washing twice with in cold PBS, non-specific binding was blocked by incubating the cells with PBS containing 1% normal horse serum, 3% BSA and 0.2% of the product sold under the registered trade mark Triton x-100. The fixed cells were then rinsed with cold PBS and incubated with specific RB1-MAB P2 supernatant diluted in PBS (1:10) for 1 hour at 37 °C. The cells were then washed sequentially with cold PBS, PBS containing 1% of the product sold under the registered trade mark Triton x-100 and finally PBS.

The specific binding of the RB1 antibody to the cells was visualized by incubation of the cells with biotinylated anti-mouse IgG antibody (diluted 1:200 in PBS) for 1 hr at 37 °C. After washing the cells three times with cold PBS, the cells were incubated with ABC (avidin:biotin complex) reagent at 37 °C for 30 min., washed with cold PBS and then incubated with freshly prepared DAB (3,3'-Diaminobenzidine tetrahydrochloride) solution at room temperature for 4 min. The DAB solution is prepared by dissolving 6mg of DAB in 10 ml. of 0.05 M Tris-HCl, pH 7.6, containing 0.3% sodium azide and 101 of hydrogen peroxide. The cells were then washed with distilled water and the stained cells analysed by microscopic examination. The results are shown on Figures 3A and 3B. The monoclonal antibody RB1-MAb20 stained all cells of the SW613 cell line known to be positive for the RB1 gene (Figure 3a). No antigen-antibody complex formation was detected in cultured cells of the RB negative cell line MDA-MB468 (Figure 3B).

The ability of the RB1 antibodies of the present invention to detect the absence of the expression of the RB1 protein in breast tumor tissue was also examined. The staining procedure can be utilized on tissue sections prepared by any of a number of conventional techniques known to those skilled in the art. For instance, the immunostaining procedures utilized to detect the presence or absence of RB1 protein in breast tissue may be utilized on paraffin sections or on frozen sections. Paraffin embedded tissue sections were deparaffinized and hydrated by contacting sequentially with xylene for 5 min., two changes of 100% ethanol for 5 min. each, 70% ethanol for 5 min. and 50% ethanol. They were then rinse for 5 minutes in PBS. The sections were the incubated for 30 minutes with PBS containing 10% normal goat serum. The sections were then incubated overnight at 4°C with RB1 antibody diluted 1:80 in PBS. After washing the tissue sections for 10 minutes in buffer, they were incubated for 60 minutes with diluted biotinylated second antibody solution. The second antibody is an antibody directed against mouse IgG. Use of the detectable label on a second antibody allows the use of unlabeled RB1 antibody in the histochemical procedure. After the tissue sections were washed for 10 minutes in buffer, the sections were incubated for 30 minutes with the ABC reagent. After the tissue sections were washed for 10 minutes in PBS, the sections were incubated for 10-20 minutes in peroxidase substrate solution (DAB). After washing the tissue sections for 5 minutes in water, they were counterstained with Mayer's haemalum and mounted prior to microscopic analysis.

In one preferred embodiment, breast tissue biopsy frozen sections (6-8 µm) were cut in a cryostat at -20°C. and post-fixed in acetone for two minutes at room temperature followed by treatment with Periodate-lysine-paraformaldehyde, pH 7.4, for eight minutes at 4°C. After blocking non-specific binding with non-immune swine serum (1:5 diluted in phosphate buffered saline, pH 7.2; PBS) for ten minutes, the tissue sections were incubated with RB1-Ab 20 (diluted 1:100 in PBS) for 60 minutes at room temperature, then rinsed. Biotinylated swine anti-rabbit immunoglobulin antiserum was then applied, followed by avidin-biotin-peroxidase complex. Peroxidase was localized using the diaminobenzidine-hydrogen peroxide reaction. Nuclei were briefly counter-stained with Mayer's haemalum.

It is evident that the fixation methods may be varied without affecting the scope or concept of the present invention. Any fixation method may be used which is suitable to preserve the RB1 protein in situ, and allow entry of the RB1 antibody to the site of the RB1 protein. It is also necessary that the fixation procedure not interfere with the binding of the RB1 antibody to the RB1 antigen.

## Example 3

### Correlation of Breast Carcinoma Grade and Stage With Decreased Detection of RB1 Protein

A major problem with interpreting the DNA probe data obtained using tumor biopsies is the heterogeneity of the tissue sample used. While it is possible to get an indication of the percentage of tumor cells within a biopsy by standard histological analysis, it is difficult to be certain of the proportion of tumor cells within the sample used for the preparation of DNA. The majority of samples studied have less than 50% tumor cells, the remainder comprising stromal cells, lymphocytes and others. A deletion of one RB1 allele in 50% of tumor cells may therefore only result in an overall reduction in hybridisation signal of 12.5%. It is therefore possible that a proportion of tumor biopsies with this level of RB1 loss are overlooked by DNA probe analysis because of the limitations of the techniques.

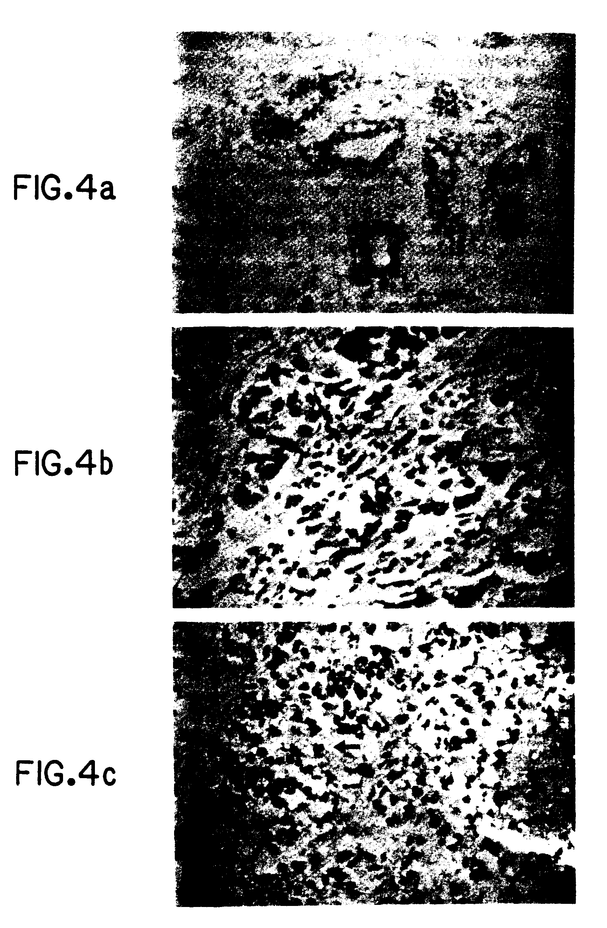

Immunohistochemical detection utilizing the RB1 antibodies of the present invention in tumor tissue sections provided a more sensitive assay of the presence or absence of RB1 protein. Staining within breast tissue, including normal breast, benign fibrocystic breast and fibroadenoma samples using the antibody RB1-Ab20 demonstrated uniform staining of all epithelial cells within the breast with nuclear staining predominating. Figure 4a demonstrates the nuclear and cytoplasmic staining pattern of epithelium of normal breast lobule (open arrow). Myoepithelial cells and stromal cells are not reactive with RB1-Ab20 and thus appear negative (closed arrow). The nuclear localization of the RB1 protein is consistent with findings of a stretch of amino acids (PKKK or Pro-Lys-Lys-Lys) characteristic of the nuclear localization signal found in nuclear proteins such as SV 40 large T antigen. Thus, all breast epithelial cells normally express RB1 to give clearly detectable levels of protein.

Of 77 carcinoma samples examined by DNA analysis for RB1 organisation, immunohistochemical analysis as described in Example 2 was carried out on 56. In 40 of these carcinoma samples, all tumor cells demonstrated nuclear reactivity, with slight variation in staining intensity. Figure Rb demonstrates a typical staining pattern of those carcinomas in which essentially all tumor cell nuclei react (arrow) with minor variations in staining intensity. Surrounding stromal cells (S) show no evidence of reactivity with the RB1-20 antibody. In the remaining 16 carcinomas, there were variable proportions of unstained tumor cells. Eleven of these had been shown to have an alteration to the RB1 gene (see Table 1), the proportion of stained tumor cells ranging from below 5% in the tumor sample with the most marked loss of RB1 (410Ca, see Figure 4c) to around 95% of cells reactive in another case. In Figure 4c, the staining pattern of a tumor section from patient 410 demonstrates that a small group of tumor cells react with the RB1 antibody (arrow) while the rest of the tumor cells are negative. This was a major area of staining within the carcinoma. In the majority of carcinomas there was no clear relationship between the proportion of tumor cells stained and the level of loss or rearrangement to RB1. The distribution of unreactive nuclei was either as small groups or islands of tumor cells (i.e. clustering), or as cells scattered throughout the tumor. The latter pattern predominated in those tumors showing minimum loss of reactivity.

Only one carcinoma (602Ca) with a clear alteration to RB1 showed no evidence of loss of reactivity to RB1-Ab20. Analysis of DNA from this tumor revealed both a deletion using one RB1 DNA probe and a rearrangement (using a second RB1 DNA probe). It seems likely that these two alterations therefore involve the same allele, allowing the generation of a normal RB1 protein from the normal allele.

In 5 samples where there were no detectable alterations to RB1 gene, there were tumor cells within the sample that lacked detectable RB1 protein, i.e., were unstained. In 4 of these cases, 5% or less of the total number of tumor cells within the section were unstained, but in one case, 50% of the cells were negative for RB1 protein (see Table 1). These samples bring the percentage of tumors with at least some cells negative for RB1 expression to 29%.

Table 1.
Details of the patients with breast carcinoma with demonstrable RB1 gene alterations or loss of expression.

| Patient No[1]. | Grade | Stage | RB1 gene alteration[2] | Percentage of expressing cells |
|---|---|---|---|---|
| 317 | III | 3 | - | 95 |
| 346 | III | 4 | - | 60 |
| 357 | III | 2 | - | 95 |
| 360 | II | 2 | - | 50 |
| 370 | III | nk | - | nk |
| 383 | III | 2 | - | 60 |
| 388 | III | 3 | - | 75 |
| 395 | III | 1 | - | 50 |
| 410 | III | 2 | - | 5 |
| 424 | II | 2 | - | 85 |
| 432 | nk | nk | - | 70 |
| 434 | II | 2 | - | 95 |
| 446 | III | 3 | - | 95 |
| 510 | III | 3 | - | 25 |
| 601 | III | nk[4] | - | 95 |
| 602 | III | * | R | 100 |
| 622 | III | nk[5] | R | 50 |
| 628 | II | nk[5] | - | 25 |
| FY | II | nk[4] | - | nk |
| FW(tub) | I | nk | - | nk |

[1]All samples are infiltrating ductal carcinomas unless otherwise indicated. Tub, tubular carcinoma.

[2]All samples show normal RB1 genomic organisation except those indicated. -, deletion of RB1; R, rearrangement of RB1.

[3]Figures in this column indicate the percentage of tumor cells within the section which react with an RB1-specific antibody.

[4]Stage of the disease not known, but nodes were not involved.

[5]Stage of disease not known, but node-positive.

*Patient had a previous history of carcinoma of the breast (both breasts).

Taken together, the data obtained on RB1 expression in primary breast tumor cells show that not only are there detectable losses of RB1 DNA in the tumor cells, but also a loss of function as measured by the expression of RB1 protein. In addition, the use of the RB1 antibody to detect RB1 protein within tissue sections has shown that detection of loss of RB1 function is more frequent than is detectable gene alteration. RB1 antibody staining of tissue sections in this situation is therefore of great value.

Utilizing the antibodies of the present invention, the alterations to RB1 gene and gene function were correlated with the particular tumor type, as well as the grade and stage of the tumor. A detectable gene deletion was observed in only one grade I tumor (a tubular carcinoma), and in no stage I tumors. In general, the frequency of genetic changes to RB1 increased with poorer differentiation of the tumor, and with the degree of spread (Table 2). Likewise loss of RB1 protein expression in the tumor cells also correlated with more advanced, less well differentiated tumors (Table 2). Although neither gene alteration nor loss of RB1 protein expression correlate with poor short-term (less than 24 months) prognosis, since alteration or deletion of the RB1 gene does correlate with advanced disease, a correlation with disease-free interval or five-year survival may become apparent when longer term follow-up data are available.

Table 2

| Distribution of the frequency of loss of expression compared to histopathological grade or to spread of the disease. | | |
|---|---|---|
| Grade[1] | Percentage of tumors with altered gene | Percentage of tumors with loss of expression |
| I | 10 (n = 10) | 0 (n = 5) |
| II | 17 (n = 30) | 19 (n = 21) |
| III | 32 (n = 25) | 48 (n = 23) |
| Stage[2] | | |
| 1 | 0 (n = 9) | 11 (n = 9) |
| 2 | 33 (n = 15) | 46 (n = 13) |
| 3 | 29 (n = 7) | 80 (n = 5) |
| 4 | 50 (n = 2) | 50 (n = 2) |

Grade I tumors are the most, and grade III the least, well differentiated tumors. A disease is stage 1 if only a localized tumor is prepared; stage 2, if a tumor is present and involvement of local lymph nodes is detected. Stage 3 indicates the presence of a large tumor with skin tethering (local invasion) and stage 4 disease, a tumor plus distant metastases.

The data reported here, indicate that the loss of RB1 function is important in the progression of human breast carcinoma. Evidence from retinoblastoma, osteosarcoma and fibrosarcoma patients, indicates that loss of function of the gene is of crucial importance for the genesis of the tumor.

One skilled in the art will readily appreciate the present invention as well adapted to carry out the object and obtain the ends and advantage mentioned, as well as those inherent therein. The antibodies, methods, procedures and techniques described herein are presented as representative of the preferred embodiments, or intended to be exemplarly and not intended as limitations on the scope of the present invention.

**Claims**

**Claims for the following Contracting States : AT, BE, CH, DE, DK, FR, GB, IT, LI, LU, NL, SE**

1. An antibody having specific binding affinity to the RB1 protein product of the retinoblastoma gene produced by immunising a host animal with a peptide located at or near the c-terminus of the RB1 protein sequence.

2. An antibody as claimed in claim 1, wherein the sequence of said peptide is selected from:

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$- Cys-Glu-Glu-Ile-Tyr-Leu-Lys-Asp-Lys-Asp-Leu-Asp-Ala-Arg-Leu-Phe-Leu-Asp-His-Asp-Lys,

and

$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys.

3. A murine hybridoma which produces a monoclonal antibody specifically immunoreactive with the RB1 protein and having specific binding affinity to a peptide sequence selected from:

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$- Cys-Glu-Glu-Ile-Tyr-Leu-Lys-Asp-Lys-Asp-Leu-Asp-Ala-Arg-Leu-Phe-Leu-Asp-His-Asp-Lys,

and

$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys.

4. A hybridoma as claimed in claim 3, deposited as ATCC HB 10093, wherein the antibody is RB1-MAB P2.

5. The monoclonal antibody producible by the hybridoma of claim 3 or claim 4.

6. A method of detecting the absence of the RB1 protein in tumor cells, comprising the steps of:
   preparing tissue sections from a tumor;
   incubating the antibody of any of claims 1, 2 or 5 with said prepared tissue sections; and
   detecting the absence of specific binding of said antibody to said tissue sections.

7. A method as claimed in claim 6 for detecting subpopulations of cells deficient in the RB1 protein.

8. A method as claimed in claim 7 for predicting the prognosis for cancer patients comprising the determination of the number of tumor cells deficient in the RB1 protein.

9. A method as claimed in claim 8, wherein said tissues are from tumors selected from the group consisting of carcinoma of the breast, carcinoma of the ovary, carcinoma of the bladder, carcinoma of the lung, carcinoma of the cervix and soft tissue sarcoma.

10. A method as claimed in claim 8, wherein said tumors are selected from the group consisting of retinoblastoma, osteosarcoma, or fibrosarcoma.

**11.** A method as claimed in claims 8 to 10 for diagnoses and prognoses for a cancer patient wherein said tissue sections are obtained by biopsing a tumor.

**Claims for the following Contracting State : ES**

**1.** Process for producing an antibody having specific binding affinity to the RB1 protein product of the retinoblastoma gene comprising immunising a host animal with a peptide located at or near the c-terminus of the RB1 protein sequence.

**2.** Process as claimed in claim 1, wherein the sequence of said peptide is selected from:

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$- Cys-Glu-Glu-Ile-Tyr-Leu-Lys-Asp-Lys-Asp-Leu-Asp-Ala-Arg-Leu-Phe-Leu-Asp-His-Asp-Lys,

and

$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys.

**3.** Process for producing a murine hybridoma which produces a monoclonal antibody specifically immunoreactive with the RB1 protein and having specific binding affinity to a peptide sequence selected from:

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$- Cys-Glu-Glu-Ile-Tyr-Leu-Lys-Asp-Lys-Asp-Leu-Asp-Ala-Arg-Leu-Phe-Leu-Asp-His-Asp-Lys,

and

$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys

comprising fusion between myeloma cells and spleen cells from immunised donors.

**4.** Process as claimed in claim 3, wherein the hybridoma is deposited as ATCC HB 10093 and the antibody is RB1-MAB P2.

**5.** Process for producing a monoclonal antibody comprising the use of a murine hybridoma which produces a monoclonal antibody specifically immunoreactive with the RB1 protein and having specific binding affinity to a peptide sequence selected from:

$H_2N-$ Cys-Arg-Met-Gln-Lys-Gln-Lys-Met-Asn-Asp-Ser-Met-Asp-Thr-Ser-Asn-Lys-Glu-Glu-Lys,

$H_2N-$ Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N-$ Cys-Glu-Glu-Ile-Tyr-Leu-Lys-Asp-Lys-Asp-Leu-Asp-Ala-Arg-Leu-Phe-Leu-Asp-His-Asp-Lys,

and

$H_2N-$ Cys-Lys-Pro-Leu-Lys-Lys-Leu-Phe-Arg-Asp-Ile-Glu-Gly-Ser-Asp-Glu-Ala-Asp-Gly-Ser-Lys.

**6.** Process as claimed in claim 5, wherein the hybridoma is deposited as ATCC HB 10093 and the antibody is RB1 - MAB P2.

**7.** A method of detecting the absence of the RB1 protein in tumor cells, comprising the steps of:
preparing tissue sections from a tumor;
incubating an antibody having specific binding affinity to the RB1 protein product of the retinoblastoma gene with said prepared tissue sections; and
detecting the absence of specific binding of said antibody to said tissue sections.

**8.** A method as claimed in claim 7 for detecting subpopulations of cells deficient in the RB1 protein.

**9.** A method as claimed in claim 8 for predicting the prognosis for cancer patients comprising the determination of the number of tumor cells deficient in the RB1 protein.

**10.** A method as claimed in claim 9, wherein said tissues are from tumors selected from the group consisting of carcinoma of the breast, carcinoma of the ovary, carcinoma of the bladder, carcinoma of the lung, carcinoma of the cervix and soft tissue sarcoma.

**11.** A method as claimed in claim 9, wherein said tumors are selected from the group consisting of retinoblastoma, osteosarcoma, or fibrosarcoma.

**12.** A method as claimed in claims 9 to 11 for diagnoses and prognoses for a cancer patient wherein said tissue sections are obtained by biopsing a tumor.

**13.** A method as claimed in claims 8 to 12, wherein said antibody is a polyclonal antibody.

**14.** A method as claimed in claims 8 to 12, wherein said antibody is a monoclonal antibody.

**15.** A method as claimed in claims 8 to 14, wherein said antibody is the antibody producible by the process of claims 1, 2 or 5.

**16.** A method as claimed in claims 8 to 12, wherein the antibody is produced from a murine hybridoma which produces a monoclonal antibody specifically immunoreactive with the RB1 protein.

**Claims for the following Contracting State : GR**

**1.** An antibody having specific binding affinity to the RB1 protein product of the retinoblastoma gene produced by immunising a host animal with a peptide located at or near the c-terminus of the RB1 protein sequence.

2. An antibody as claimed in claim 1, wherein the sequence of said peptide is selected from:

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$- Cys-Glu-Glu-Ile-Tyr-Leu-Lys-Asp-Lys-Asp-Leu-Asp-Ala-Arg-Leu-Phe-Leu-Asp-His-Asp-Lys,

and

$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys.

3. A murine hybridoma which produces a monoclonal antibody specifically immunoreactive with the RB1 protein and having specific binding affinity to a peptide sequence selected from:

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$- Cys-Glu-Glu-Ile-Tyr-Leu-Lys-Asp-Lys-Asp-Leu-Asp-Ala-Arg-Leu-Phe-Leu-Asp-His-Asp-Lys,

and

$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys.

4. A hybridoma as claimed in claim 3, deposited as ATCC HB 10093 wherein the antibody is RB1-MAB P2.

5. The monoclonal antibody producible by the hybridoma of claim 3 or claim 4.

6. Process for producing an antibody having specific binding affinity to the RB1 protein product of the retinoblastoma gene comprising immunising a host animal with a peptide located at or near the c-terminus of the RB1 protein sequence.

**7.** Process as claimed in claim 6, wherein the sequence of said peptide is selected from:

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$- Cys-Glu-Glu-Ile-Tyr-Leu-Lys-Asp-Lys-Asp-Leu-Asp-Ala-Arg-Leu-Phe-Leu-Asp-His-Asp-Lys,

and

$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys.

**8.** Process for producing a murine hybridoma which produces a monoclonal antibody specifically immunoreactive with the RB1 protein and having specific binding affinity to a peptide sequence selected from:

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$- Cys-Glu-Glu-Ile-Tyr-Leu-Lys-Asp-Lys-Asp-Leu-Asp-Ala-Arg-Leu-Phe-Leu-Asp-His-Asp-Lys,

and

$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys.

comprising fusion between myeloma cells and spleen cells from immunised donors.

**9.** Process as claimed in claim 8, wherein the hybridoma is deposited as ATCC HB 10093 and the antibody is RB1-MAB P2.

**10.** Process for producing a monoclonal antibody comprising the use of a murine hybridoma which produces a monoclonal antibody specifically immunoreactive with the RB1 protein and having specific binding affinity to a peptide sequence selected from:

$H_2N-$ Cys-Arg-Met-Gln-Lys-Gln-Lys-Met-Asn-Asp-Met-Asp-Thr-Ser-Asn-Lys-Glu-Glu-Lys,

$H_2N-$ Cys-Arg-Thr-Pro-Arg-Arg-Gly-Gln-Asp-Arg-Ala-Arg-Ile-Ala-Lys-Gln-Leu-Glu-Asp-Asp,

$H_2N-$ Cys-Glu-Glu-Ile-Tyr-Leu-Lys-Asp-Lys-Asp-Asp-Ala-Arg-Leu-Phe-Leu-Asp-His-Asp-Lys,

and

$H_2N-$ Cys-Lys-Pro-Leu-Lys-Lys-Leu-Phe-Arg-Asp-Glu-Gly-Ser-Asp-Glu-Ala-Asp-Gly-Ser-Lys.

**11.** Process as claimed in claim 10, wherein the hybridoma is deposited as ATCC HB 10093 and the antibody is RB1 - MAB P2.

**12.** A method of detecting the absence of the RB1 protein in tumor cells, comprising the steps of:
preparing tissue sections from a tumor;
incubating an antibody having specific binding affinity to the RB1 protein product of the retinoblastoma gene with said prepared tissue sections; and
detecting the absence of specific binding of said antibody to said tissue sections.

**13.** A method as claimed in claim 12 for detecting subpopulations of cells deficient in the RB1 protein.

**14.** A method as claimed in claim 13 for predicting the prognosis for cancer patients comprising the determination of the number of tumor cells deficient in the RB1 protein.

**15.** A method as claimed in claim 14, wherein said tissues are from tumors selected from the group consisting of carcinoma of the breast, carcinoma of the ovary, carcinoma of the bladder, carcinoma of the lung, carcinoma of the cervix and soft tissue sarcoma.

**16.** A method as claimed in claim 14, wherein said tumors are selected from the group consisting of retinoblastoma, osteosarcoma, or fibrosarcoma.

**17.** A method as claimed in claims 14 to 16 for diagnoses and prognoses for a cancer patient wherein said tissue sections are obtained by biopsing a tumor.

**18.** A method as claimed in claims 13 to 17, wherein said antibody is a polyclonal antibody.

**19.** A method as claimed in claims 13 to 17, wherein said antibody is a monoclonal antibody.

**20.** A method as claimed in claims 13 to 19, wherein said antibody is the antibody producible by the process of claims 6, 7 or 10.

**21.** A method as claimed in claims 13 to 17, wherein the antibody is produced from a murine hybridoma which produces a monoclonal antibody specifically immunoreactive with the RB1 protein.

**Patentansprüche**
**Patentansprüche für folgende Vertragsstaaten : AT, BE, CH, DE, DK, FR, GB, IT, LI, LU, NL, SE**

**1.** Antikörper mit spezifischer Bindungsaffinität für das RB1-Protein-Produkt des Retinoblastom-Gens, der hergestellt wird, indem ein Wirtstier mit einem Peptid immunisiert wird, das am oder in der Nähe des C-Terminus der RB1-Protein-Sequenz lokalisiert ist.

2. Antikörper nach Anspruch 1, wobei die Sequenz des Peptids ausgewählt ist aus:

H2N- Cys-Arg-Met-Gln-Lys-Gln-Lys-Met-Asn-Asp-Ser-Met-Asp-Thr-Ser-Asn-Lys-Glu-Glu-Lys,

H2N- Cys-Arg-Thr-Pro-Arg-Arg-Gly-Gln-Asp-Arg-Ser-Ala-Arg-Ile-Ala-Lys-Gln-Leu-Glu-Asp-Asp,

H2N- Cys-Glu-Glu-Ile-Tyr-Leu-Lys-Asp-Lys-Asp-Leu-Asp-Ala-Arg-Leu-Phe-Leu-Asp-His-Asp-Lys,

und

H2N- Cys-Lys-Pro-Leu-Lys-Lys-Leu-Phe-Arg-Asp-Ile-Glu-Gly-Ser-Asp-Glu-Ala-Asp-Gly-Ser-Lys.

3. Maus-Hybridom, das einen monoklonalen Antikörper bildet, der mit dem RB1-Protein spezifisch immunreaktiv ist und spezifische Bindungsaffinität für eine Peptid-Sequenz aufweist, die ausgewählt ist aus:

H2N- Cys-Arg-Met-Gln-Lys-Gln-Lys-Met-Asn-Asp-Ser-Met-Asp-Thr-Ser-Asn-Lys-Glu-Glu-Lys,

H2N- Cys-Arg-Thr-Pro-Arg-Arg-Gly-Gln-Asp-Arg-Ser-Ala-Arg-Ile-Ala-Lys-Gln-Leu-Glu-Asp-Asp,

H2N- Cys-Glu-Glu-Ile-Tyr-Leu-Lys-Asp-Lys-Asp-Leu-Asp-Ala-Arg-Leu-Phe-Leu-Asp-His-Asp-Lys,

und

H2N- Cys-Lys-Pro-Leu-Lys-Lys-Leu-Phe-Arg-Asp-Ile-Glu-Gly-Ser-Asp-Glu-Ala-Asp-Gly-Ser-Lys.

4. Hybridom nach Anspruch 3, das als ATCC HB 10093 hinterlegt ist, bei dem es sich bei dem Antikörper um RB1-MAB P2 handelt.

5. Monoklonaler Antikörper, der mit dem Hybridom nach Anspruch 3 oder Anspruch 4 herstellbar ist.

6. Verfahren zum Nachweis des Fehlens des RB1-Proteins in Tumorzellen, bei dem:
Gewebeschnitte eines Tumors präpariert werden,
der Antikörper nach einem der Ansprüche 1, 2 oder 5 mit den präparierten Gewebeschnitten inkubiert wird; und
das Fehlen der spezifischen Bindung des Antikörpers an die Gewebeschnitte nachgewiesen wird.

7. Verfahren nach Anspruch 6 zum Nachweis von Unterpopulationen von Zellen, denen das RB1-Protein fehlt.

8. Verfahren nach Anspruch 7 zum Stellen der Prognose für Krebspatienten, bei dem die Anzahl Tumorzellen ermittelt wird, denen das RB1-Protein fehlt.

9. Verfahren nach Anspruch 8, bei dem die Gewebe von Tumoren stammen, die aus der aus dem Brustcarcinom, dem Eierstockcarcinom, dem Blasencarcinom, dem Lungencarcinom, dem Gebärmutterhalscarcinom und dem Weichteilsarkom bestehenden Gruppe ausgewählt sind.

10. Verfahren nach Anspruch 8, bei dem die Tumoren aus der aus dem Retinoblastom, dem Osteosarkom oder dem Fibrosarkom bestehenden Gruppe ausgewählt sind.

11. Verfahren nach den Ansprüchen 8 bis 10 zur Diagnose und Prognose für einen Krebspatienten, bei dem die Gewebeschnitte erhalten werden, indem ein Tumor biopsiert wird.

**Patentansprüche für folgenden Vertragsstaat : ES**

1. Verfahren zur Herstellung eines Antikörpers mit spezifischer Bindungsaffinität für das RB1-Protein-Produkt des Retinoblastom-Gens, bei dem ein Wirtstier mit einem Peptid immunisiert wird, das am oder in der Nähe des C-Terminus der RB1-Protein-Sequenz lokalisiert ist.

2. Verfahren nach Anspruch 1, wobei die Sequenz des Peptids ausgewählt ist aus:

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$- Cys-Glu-Glu-Ile-Tyr-Leu-Lys-Asp-Lys-Asp-Leu-Asp-Ala-Arg-Leu-Phe-Leu-Asp-His-Asp-Lys,

und

$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys.

3. Verfahren zur Herstellung eines Maus-Hybridoms, das einen monoklonalen Antikörper bildet, der mit dem RB1-Protein spezifisch immunreaktiv ist und spezifische Bindungsaffinität für eine Peptid-Sequenz aufweist, die ausgewählt ist aus:

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$- Cys-Glu-Glu-Ile-Tyr-Leu-Lys-Asp-Lys-Asp-Leu-Asp-Ala-Arg-Leu-Phe-Leu-Asp-His-Asp-Lys,

und

$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys

bei dem Myelomzellen und Milzzellen von immunisierten Donoren fusioniert werden.

4. Verfahren nach Anspruch 3, wobei das Hybridom als ATCC HB 10093 hinterlegt ist und es sich bei dem Antikörper um RB1-MAB P2 handelt.

5. Verfahren zur Herstellung eines monoklonalen Antikörpers, bei dem ein Maus-Hybridom verwendet wird, das einen monoklonalen Antikörper bildet, der mit dem RB1-Protein spezifisch immunreaktiv ist und spezifische Bindungsaffinität für eine Peptid-Sequenz aufweist, die ausgewählt ist aus:

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$- Cys-Glu-Glu-Ile-Tyr-Leu-Lys-Asp-Lys-Asp-Leu-Asp-Ala-Arg-Leu-Phe-Leu-Asp-His-Asp-Lys,

und

$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys.

6. Verfahren nach Anspruch 5, wobei das Hybridom als ATCC HB 10093 hinterlegt ist und es sich bei dem Antikörper um RB1-MAB P2 handelt.

7. Verfahren zum Nachweis des Fehlens des RB1-Proteins in Tumorzellen, bei dem:
Gewebeschnitte eines Tumors präpariert werden;
ein Antikörper mit spezifischer Bindungsaffinität für das RB1-Protein-Produkt des Retinoblastom-Gens mit den präparierten Gewebeschnitten inkubiert wird; und
das Fehlen der spezifischen Bindung des Antikörpers an die Gewebeschnitte nachgewiesen wird.

8. Verfahren nach Anspruch 7 zum Nachweis von Unterpopulationen von Zellen, denen das RB1-Protein fehlt.

9. Verfahren nach Anspruch 8 zum Stellen der Prognose für Krebspatienten, bei dem die Anzahl Tumorzellen ermittelt wird, denen das RB1-Protein fehlt.

10. Verfahren nach Anspruch 9, bei dem die Gewebe von Tumoren stammen, die aus der aus dem Brustcarcinom, dem Eierstockcarcinom, dem Blasencarcinom, dem Lungencarcinom, dem Gebärmutterhalscarcinom und dem Weichteilsarkom bestehenden Gruppe ausgewählt sind.

11. Verfahren nach Anspruch 9, bei dem die Tumoren aus der aus dem Retinoblastom, dem Osteosarkom oder dem Fibrosarkom bestehenden Gruppe ausgewählt sind.

12. Verfahren nach den Ansprüchen 9 bis 11 zur Diagnose und Prognose für einen Krebspatienten, bei dem die Gewebeschnitte erhalten werden, indem ein Tumor biopsiert wird.

13. Verfahren nach den Ansprüchen 8 bis 12, wobei es sich bei dem Antikörper um einen polyklonalen Antikörper handelt.

14. Verfahren nach den Ansprüchen 8 bis 12, wobei es sich bei dem Antikörper um einen monoklonalen Antikörper handelt.

**15.** Verfahren nach den Ansprüchen 8 bis 14, wobei es sich bei dem Antikörper um den Antikörper handelt, der mit dem Verfahren nach den Ansprüchen 1, 2 oder 5 herstellbar ist.

**16.** Verfahren nach den Ansprüchen 8 bis 12, bei dem der Antikörper von einem Maus-Hybridom hergestellt wird, das einen monoklonalen Antikörper bildet, der mit dem RB1-Protein spezifisch immunreaktiv ist.

**Patentansprüche für folgenden Vertragsstaat : GR**

**1.** Antikörper mit spezifischer Bindungsaffinität für das RB1-Protein-Produkt des Retinoblastom-Gens, der hergestellt wird, indem ein Wirtstier mit einem Peptid immunisiert wird, das am oder in der Nähe des C-Terminus der RB1-Protein-Sequenz lokalisiert ist.

**2.** Antikörper nach Anspruch 1, wobei die Sequenz des Peptids ausgewählt ist aus:

$H_2N$- Cys-Arg-Met-Gln-Lys-Gln-Lys-Met-Asn-Asp-Ser-Met-Asp-Thr-Ser-Asn-Lys-Glu-Glu-Lys,
$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,
$H_2N$- Cys-Glu-Glu-Ile-Tyr-Leu-Lys-Asp-Lys-Asp-Leu-Asp-Ala-Arg-Leu-Phe-Leu-Asp-His-Asp-Lys,
und
$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys.

**3.** Maus-Hybridom, das einen monoklonalen Antikörper bildet, der mit dem RB1-Protein spezifisch immunreaktiv ist und spezifische Bindungsaffinität für eine Peptid-Sequenz aufweist, die ausgewählt ist aus:

$H_2N$- Cys-Arg-Met-Gln-Lys-Gln-Lys-Met-Asn-Asp-Ser-Met-Asp-Thr-Ser-Asn-Lys-Glu-Glu-Lys,
$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,
$H_2N$- Cys-Glu-Glu-Ile-Tyr-Leu-Lys-Asp-Lys-Asp-Leu-Asp-Ala-Arg-Leu-Phe-Leu-Asp-His-Asp-Lys,
und
$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys.

**4.** Hybridom nach Anspruch 3, das als ATCC HB 10093 hinterlegt ist, bei dem es sich bei dem Antikörper um RB1-MAB P2 handelt.

**5.** Monoklonaler Antikörper, der mit dem Hybridom nach Anspruch 3 oder Anspruch 4 herstellbar ist.

6. Verfahren zur Herstellung eines Antikörpers mit spezifischer Bindungsaffinität für das RB1-Protein-Produkt des Retinoblastom-Gens, bei dem ein Wirtstier mit einem Peptid immunisiert wird, das am oder in der Nähe des C-Terminus der RB1-Protein-Sequenz lokalisiert ist.

7. Verfahren nach Anspruch 6, wobei die Sequenz des Peptids ausgewählt ist aus:

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$- Cys-Glu-Glu-Ile-Tyr-Leu-Lys-Asp-Lys-Asp-Leu-Asp-Ala-Arg-Leu-Phe-Leu-Asp-His-Asp-Lys,

und

$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys.

8. Verfahren zur Herstellung eines Maus-Hybridoms, das einen monoklonalen Antikörper bildet, der mit dem RB1-Protein spezifisch immunreaktiv ist und spezifische Bindungsaffinität für eine Peptid-Sequenz aufweist, die ausgewählt ist aus:

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$- Cys-Glu-Glu-Ile-Tyr-Leu-Lys-Asp-Lys-Asp-Leu-Asp-Ala-Arg-Leu-Phe-Leu-Asp-His-Asp-Lys,

und

$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys

bei dem Myelomzellen und Milzzellen von immunisierten Donoren fusioniert werden.

9. Verfahren nach Anspruch 8, wobei das Hybridom als ATCC HB 10093 hinterlegt ist und es sich bei dem Antikörper um RB1-MAB P2 handelt.

10. Verfahren zur Herstellung eines monoklonalen Antikörpers, bei dem ein Maus-Hybridom verwendet wird, das einen monoklonalen Antikörper bildet, der mit dem RB1-Protein spezifisch immunreaktiv ist und spezifische Bindungsaffinität für eine Peptid-Sequenz aufweist, die ausgewählt ist aus:

$H_2N-$ Cys-Arg-Met-Gln-Lys-Gln-Lys-Met-Asn-Asp-Met-Asp-Thr-Ser-Asn-Lys-Glu-Glu-Lys,

$H_2N-$ Cys-Arg-Thr-Pro-Arg-Arg-Gly-Gln-Asp-Arg-Ala-Arg-Ile-Ala-Lys-Gln-Leu-Glu-Asp-Asp,

$H_2N-$ Cys-Glu-Glu-Ile-Tyr-Leu-Lys-Asp-Lys-Asp-Asp-Ala-Arg-Leu-Phe-Leu-Asp-His-Asp-Lys,

und

$H_2N-$ Cys-Lys-Pro-Leu-Lys-Lys-Leu-Phe-Arg-Asp-Glu-Gly-Ser-Asp-Glu-Ala-Asp-Gly-Ser-Lys.

**11.** Verfahren nach Anspruch 10, wobei das Hybridom als ATCC HB 10093 hinterlegt ist und es sich bei dem Antikörper um RB1-MAB P2 handelt.

**12.** Verfahren zum Nachweis des Fehlens des RB1-Proteins in Tumorzellen, bei dem:
Gewebeschnitte eines Tumors präpariert werden;
ein Antikörper mit spezifischer Bindungsaffinität für das RB1-Protein-Produkt des Retinoblastom-Gens mit den präparierten Gewebeschnitten inkubiert wird; und
das Fehlen der spezifischen Bindung des Antikörpers an die Gewebeschnitte nachgewiesen wird.

**13.** Verfahren nach Anspruch 12 zum Nachweis von Unterpopulationen von Zellen, denen das RB1-Protein fehlt.

**14.** Verfahren nach Anspruch 13 zum Stellen der Prognose für Krebspatienten, bei dem die Anzahl Tumorzellen ermittelt wird, denen das RB1-Protein fehlt.

**15.** Verfahren nach Anspruch 14, bei dem die Gewebe von Tumoren stammen, die aus der aus dem Brustcarcinom, dem Eierstockcarcinom, dem Blasencarcinom, dem Lungencarcinom, dem Gebärmutterhalscarcinom und dem Weichteilsarkom bestehenden Gruppe ausgewählt sind.

**16.** Verfahren nach Anspruch 14, bei dem die Tumoren aus der aus dem Retinoblastom, dem Osteosarkom oder dem Fibrosarkom bestehenden Gruppe ausgewählt sind.

**17.** Verfahren nach den Ansprüchen 14 bis 16 zur Diagnose und Prognose für einen Krebspatienten, bei dem die Gewebeschnitte erhalten werden, indem ein Tumor biopsiert wird.

**18.** Verfahren nach den Ansprüchen 13 bis 17, wobei es sich bei dem Antikörper um einen polyklonalen Antikörper handelt.

**19.** Verfahren nach den Ansprüchen 13 bis 17, wobei es sich bei dem Antikörper um einen monoklonalen Antikörper handelt.

**20.** Verfahren nach den Ansprüchen 13 bis 19, wobei es sich bei dem Antikörper um den Antikörper handelt, der mit dem Verfahren nach den Ansprüchen 6, 7 oder 10 herstellbar ist.

**21.** Verfahren nach den Ansprüchen 13 bis 17, bei dem der Antikörper von einem Maus-Hybridom hergestellt wird, das einen monoklonalen Antikörper bildet, der mit dem RB1-Protein spezifisch immunreaktiv ist.

**Revendications**
**Revendications pour les Etats contractants suivants : AT, BE, CH, DE, DK, FR, GB, IT, LI, LU, NL, SE**

1. Anticorps ayant de l'affinité de liaison spécifique sur le produit protéinique RB1 du gène de rétinoblastome produit par immunisation d'un animal hôte avec un peptide,situé à la terminaison carbonée ou près de la terminaison carbonée, de la séquence de protéine RB1.

2. Anticorps tel que revendiqué à la revendication 1, dans lequel la séquence dudit peptide est choisie parmi :

$H_2N$- Cys-Arg-Met-Gln-Lys-Gln-Lys-Met-Asn-Asp-Ser-Met-Asp-Thr-Ser-Asn-Lys-Glu-Glu-Lys,
$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,
$H_2N$ Cys-Glu-Glu-Ile-Tyr-Leu-Lys-Asp-Lys-Asp-Leu-Asp-Ala-Arg-Leu-Phe-Leu-Asp-His-Asp-Lys,
et
$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys.

3. Hybridome de souris, qui produit un anticorps monoclonal spécifiquement immunoréactif avec la protéine RB1 et qui a une affinité spécifique de liaison sur une séquence peptidique choisie parmi

$H_2N$- Cys-Arg-Met-Gln-Lys-Gln-Lys-Met-Asn-Asp-Ser-Met-Asp-Thr-Ser-Asn-Lys-Glu-Glu-Lys,
$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,
$H_2N$ Cys-Glu-Glu-Ile-Tyr-Leu-Lys-Asp-Lys-Asp-Leu-Asp-Ala-Arg-Leu-Phe-Leu-Asp-His-Asp-Lys,
et
$H_2N$- Cys-Lys-Pro-Leu-Lys-Lys-Leu-Phe-Arg-Asp-Ile-Glu-Gly-Ser-Asp-Glu-Ala-Asp-Gly-Ser-Lys.

4. Hybridome tel que revendiqué à la revendication 3, déposé sous la référence ATCC HB 10 093, dans lequel l'anticorps est RB1-MAB P2.

5. Anticorps monoclonal, pouvant être produit par l'hybridome de la revendication 3 ou de la revendication 4.

6. Procédé de détection de l'absence de protéine RBI dans des cellules d'une tumeur, le procédé comprenant les étapes consistant à :
   préparer les tranches ou coupes de tissu prélevées sur une tumeur ;
   faire incuber l'anticorps selon l'une quelconque des revendications 1, 2 ou 5 avec lesdites tranches du tissu ainsi préparées ; et
   déceler l'absence de liaison spécifique dudit anticorps sur lesdites tranches de tissu.

7. Procédé selon la revendication 6, pour déceler des sous-populations de cellules dépourvues de la protéine RBI ou présentant un manque de cette protéine.

8. Procédé selon la revendication 7 pour prédire le pronostic de cancer chez les patients, le procédé comprenant la détermination du nombre des cellules de tumeurs dépourvues de la protéine RB1.

9. Procédé selon la revendication 8, dans lequel lesdits tissus proviennent de tumeurs choisies dans l'ensemble consistant en un carcinome du sein, un carcinome des ovaires, un carcinome de la vessie, un carcinome du poumon, un carcinome du col (de l'utérus) et un sarcome de tissu mou.

10. Procédé selon la revendication 8, dans lequel les tumeurs sont choisies dans l'ensemble consistant en du rétinoblastome, de l'ostéosarcome ou du fibrosarcome.

11. Procédé selon les revendications 8 à 10 pour poser un diagnostic et un pronostic de cancer chez un patient, procédé dans lequel on obtient lesdites tranches ou coupes de tissu en effectuant un prélèvement de biopsie sur une tumeur.

**Revendications pour l'Etat contractant suivant : ES**

1. Procédé pour produire un anticorps ayant de l'affinité de liaison spécifique sur la protéine RB1 produite par le gène de rétinoblastome, ce procédé comprenant l'immunisation d'un hôte animal par un peptide situé à la terminaison carbonée ou près de la terminaison carbonée de la séquence de protéine RB1.

2. Procédé selon la revendication 1, dans lequel la séquence dudit peptide est choisie parmi

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$ Cys-Glu-Glu-Ile-Tyr-Leu-Lys-Asp-Lys-Asp-Leu-Asp-Ala-Arg-Leu-Phe-Leu-Asp-His-Asp-Lys,

et

$H_2N$- Cys-Lys-Pro-Leu-Lys-Lys-Leu-Phe, Arg-Asp-Ile-Glu-Gly-Ser-Asp-Glu-Ala-Asp-Gly-Ser-Lys.

3. Procédé pour produire un hybridome de souris produisant un anticorps monoclonal spécifiquement immunoréactif avec la protéine RB1 et ayant de l'affinité de liaison spécifique sur une séquence de peptide choisie parmi

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$ Cys-Glu-Glu-Ile-Tyr-Leu-Lys-Asp-Lys-Asp-Leu-Asp-Ala-Arg-Leu-Phe-Leu-Asp-His-Asp-Lys,

et

$H_2N$- Cys-Lys-Pro-Leu-Lys-Lys-Leu-Phe, Arg-Asp-Ile-Glu-Gly-Ser-Asp-Glu-Ala-Asp-Gly-Ser-Lys

comprenant une fusion entre des cellules de myélome et des cellules de rate provenant de donneurs immunisés.

4. Procédé selon la revendication 3, dans le cas duquel L'hybridome est déposé sous La référence ATCC HB 10 093 et l'anticorps est RB1-MAB P2.

5. Procédé pour produire un anticorps monoclonal comprenant l'utilisation d'un hybridome de souris qui produit un anticorps monoclonal spécifiquement immunoréactif avec la protéine RB1 et ayant de l'affinité de liaison spécifique sur une séquence de peptide choisie parmi

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$ Cys-Glu-Glu-Ile-Tyr-Leu-Lys-Asp-Lys-Asp-Leu-Asp-Ala-Arg-Leu-Phe-Leu-Asp-His-Asp-Lys,

et

$H_2N$- Cys-Lys-Pro-Leu-Lys-Lys-Leu-Phe, Arg-Asp-Ile-Glu-Gly-Ser-Asp-Glu-Ala-Asp-Gly-Ser-Lys.

6. Procédé selon la revendication 5, dans le cas duquel l'hybridome est déposé sous la référence ATCC HB 10 093 et l'anticorps est RB1-MAB P2.

7. Procédé pour déceler l'absence de protéine RB1 dans des cellules de tumeurs, ce procédé comprenant les étapes consistant à :

préparer des tranches ou coupes de tissu prélevées sur une tumeur ;

faire incuber un anticorps, ayant de l'affinité de liaison spécifique sur la protéine RB1 produite par le gène de rétinoblastome, avec lesdites tranches de tissu ainsi préparées ; et

déceler l'absence de liaison spécifique dudit anticorps sur lesdites tranches de tissu.

8. Procédé selon la revendication 7 pour déceler des sous-populations de cellules dépourvues de la protéine RB1 ou manquant d'une telle protéine.

9. Procédé selon la revendication 8 pour prédire le pronostic de cancer chez des patients, ce procédé comprenant la détermination du nombre de cellules de tumeurs dépourvues de la protéine RB1 ou manquant d'une telle protéine.

**10.** Procédé selon la revendication 9, dans lequel lesdits tissus sont choisis parmi les tumeurs choisies dans l'ensemble consistant en un carcinome du sein, un carcinome de l'ovaire, un carcinome de la vessie, un carcinome du poumon, un carcinome de col (de l'utérus) et un sarcome de tissu mou.

**11.** Procédé selon la revendication 9, dans lequel lesdites tumeurs sont choisies dans l'ensemble consistant en le rétinoblastome, de l'ostéosarcome ou du fibrosarcome.

**12.** Procédé selon les revendications 9 à 11 pour le diagnostic et le pronostic d'un cancer chez un patient, procédé dans lequel lesdites tranches de tissu sont obtenues par prélèvement d'une biopsie sur une tumeur.

**13.** Procédé selon les revendications 8 à 12, dans lequel ledit anticorps est un anticorps polyclonal.

**14.** Procédé selon les revendications 8 à 12, dans lequel ledit anticorps est un anticorps monoclonal.

**15.** Procédé selon les revendication 8 à 14, dans lequel ledit anticorps est l'anticorps pouvant être produit par le procédé selon les revendications 1, 2 ou 5.

**16.** Procédé selon les revendications 8 à 12, dans lequel l'anticorps est produit à partir d'un hybridome de souris qui produit un anticorps monoclonal spécifiquement immunoréactif avec la protéine RB1.

**Revendications pour l'Etat contractant suivant : GR**

**1.** Anticorps ayant une affinité de liaison spécifique sur le produit protéinique RB1 du gène de rétinoblastome, que l'on produit en immtinisant un hôte animal à l'aide d'un peptide situé à la terminaison carbonée ou près de la terminaison carbonée de la séquence de protéine RB1.

**2.** Anticorps selon la revendication 1, dans lequel la séquence dudit peptide est choisie parmi

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$ Cys-Glu-Glu-Ile-Tyr-Leu-Lys-Asp-Lys-Asp-Leu-Asp-Ala-Arg-Leu-Phe-Leu-Asp-His-Asp-Lys,

et

$H_2N$- Cys-Lys-Pro-Leu-Lys-Lys-Leu-Phe→ Arg-Asp-Ile-Glu-Gly-Ser-Asp-Glu-Ala-Asp-Gly-Ser-Lys.

**3.** Hybridome de souris, qui produit un anticorps monoclonal spécifiquement immunoréactif avec la protéine RB1 et qui a de l'affinité de liaison spécifique sur une séquence peptidique choisie parmi

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$ Cys-Glu-Glu-Ile-Tyr-Leu-Lys-Asp-Lys-Asp-Leu-Asp-Ala-Arg-Leu-Phe-Leu-Asp-His-Asp-Lys,

et

$H_2N$- Cys-Lys-Pro-Leu-Lys-Lys-Leu-Phe- Arg-Asp-Ile-Glu-Gly-Ser-Asp-Glu-Ala-Asp-Gly-Ser-Lys.

4. Hybridome selon la revendication 3, déposé sous la référence ATCC HB 10 093, dans lequel l'anticorps est RB1-MAB P2.

5. Anticorps monoclonal pouvant être produit par l'hybridome de la revendication 3 ou 4.

6. Procédé pour produire un anticorps ayant de l'affinité de liaison spécifique sur la protéine RB1 produite par le gène de rétinoblastome, ce procédé comprenant l'immunisation d'un hôte animal à l'aide d'un peptide situé à la terminaison carbonée ou près de la terminaison carbonée de la séquence de protéine RB1.

7. Procédé selon la revendication 6, dans lequel la séquence dudit peptide est choisie parmi

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$ Cys-Glu-Glu-Ile-Tyr-Leu-Lys-Asp-Lys-Asp-Leu-Asp-Ala-Arg-Leu-Phe-Leu-Asp-His-Asp-Lys,

et

$H_2N$- Cys-Lys-Pro-Leu-Lys-Lys-Leu-Phe- Arg-Asp-Ile-Glu-Gly-Ser-Asp-Glu-Ala-Asp-Gly-Ser-Lys.

8. Procédé pour produire un hybridome de souris produisant un anticorps monoclonal à immunoréactivité spécifique avec la protéine RBI et ayant de l'affinité de liaison spécifique sur une séquence peptidique choisie parmi

$H_2N$- Cys-Arg-Met-Gln-Lys-Gln-Lys-Met-Asn-Asp-Ser-Met-Asp-Thr-Ser-Asn-Lys-Glu-Glu-Lys,

$H_2N$- Cys-Arg-Thr-Pro-Arg-Arg-Gly-Gln-Asp-Arg-Ser-Ala-Arg-Ile-Ala-Lys-Gln-Leu-Glu-Asp-Asp,

$H_2N$ Cys-Glu-Glu-Ile-Tyr-Leu-Lys-Asp-Lys-Asp-Leu-Asp-Ala-Arg-Leu-Phe-Leu-Asp-His-Asp-Lys,

et

$H_2N$- Cys-Lys-Pro-Leu-Lys-Lys-Leu-Phe - Arg-Asp-Ile-Glu-Gly-Ser-Asp-Glu-Ala-Asp-Gly-Ser-Lys.

le procédé comprenant une fusion entre des cellules de myélome et des cellules de rate provenant de donneurs immunisés.

**9.** Procédé selon la revendication 8, dans le cas duquel l'hybridome est déposé sous la désignation ATCC HB 10 093 et l'anticorps est RB1-MAB P2.

**10.** Procédé pour produire un anticorps monoclonal, comprenant l'utilisation d'un hybridome de souris qui produit un anticorps monoclonal spécifiquement immunoréactif avec la protéine RB1 et ayant de l'affinité de liaison spécifique sur une séquence de peptide choisie parmi

$H_2N$- Cys-Arg-Met-Gln-Lys-Gln-Lys-Met-Asn-Asp-Met-Asp-Thr-Ser-Asn-Lys-Glu-Glu-Lys,

$H_2N$- Cys-Arg-Thr-Pro-Arg-Arg-Gly-Gln-Asp-Arg-Ala-Arg-Ile-Ala-Lys-Gln-Leu-Glu-Asp-Asp,

$H_2N$ Cys-Glu-Glu-Ile-Tyr-Leu-Lys-Asp-Lys-Asp-Asp-Ala-Arg-Leu-Phe-Leu-Asp-His-Asp-Lys,

et

$H_2N$- Cys-Lys-Pro-Leu-Lys-Lys-Leu-Phe Arg-Asp-Glu-Gly-Ser-Asp-Glu-Ala-Asp-Gly-Ser-Lys.

**11.** Procédé selon la revendication 10, dans le cas duquel l'hybridome est déposé sous la désignation ATCC HB 10 093 et l'anticorps est RB1-MAB P2.

**12.** Procédé pour déceler l'absence de protéines RB1 dans des cellules de tumeurs, comprenant les étapes consistant à :

préparer des tranches de tissu prélevées sur une tumeur ;

faire incuber un anticorps, ayant de l'affinité de liaison spécifique sur la protéine RB1 produite par le gène de rétinoblastome avec lesdites tranches de tissu ainsi préparées ; et

déceler l'absence de liaison spécifique dudit anticorps sur lesdites tranches de tissu.

**13.** Procédé selon la revendication 12 pour déceler des sous-populations de cellules dépourvues de La protéine RB1.

**14.** Procédé selon la revendication 13 pour prédire le pronostic de cancer pour des patients, le procédé comprenant la détermination du nombre de cellules de tumeurs dépourvues de la protéine RB1.

**15.** Procédé selon la revendication 14, dans lequel lesdits tissus sont des tissus provenant de tumeurs choisies dans l'ensemble consistant en un carcinome du sein, un carcinome de l'ovaire, un carcinome de la vessie, un carcinome du poumon, un carcinome du col (de l'utérus) et du sarcome de tissu mou.

**16.** Procédé selon la revendication 14, dans le cas duquel les tumeurs sont choisies dans l'ensemble consistant en du rétinoblastome, de l'ostéosarcome ou du fibrosarcome.

**17.** Procédé selon les revendications 14 à 16 pour le diagnostic et le pronostic d'un cancer chez un patient, procédé dans lequel on obtient lesdites tranches de tissu par prélèvement d'une biopsie sur une tumeur.

**18.** Procédé selon les revendications 13 à 17, dans lequel ledit anticorps est un anticorps polyclonal.

**19.** Procédé selon la revendication 13 à 17, dans lequel ledit anticorps est un anticorps monoclonal.

**20.** Procédé selon les revendications 13 à 19, dans lequel ledit anticorps est l'anticorps pouvant être produit par le procédé selon les revendications 6, 7 ou 10.

**21.** Procédé selon les revendications 13 à 17, dans lequel l'anticorps est produit à partir d'un hybridome de souris produisant un anticorps monoclonal spécifiquement immunoréactif avec la protéine RB1.

FIG. 1-1

5' GATTAAATAAGACAATTATGTAAGGTGGCCAGCACAGTTCCTGGTACATAGTAAATGTCAGGCCTGCCTGACAGACTTCTATTCAGCAGCTACTGCTCCC -801

CTGAAAATCTTCCTCAGACGTTTCCACGGTGCTTCCCGTTCTTACACCACTACAATCCTTTATTACACTACTATCCGTTCATTCCCCACAGCTCCCTCCC -701

TTCCTTTCCCTAACCAGTGATCCCAAAAGGCCAGCAAGTGTCTAACATTTTCTATCTTCTAAGTGACTGGTAAAGTTCCGCACCTATCAGCGCTCCAAGT -601

Sp I

TTGTTTTTGTTTTGGCCGACTTTGCAAAACGGATTGGCGGGATGAGAGGTGGGGGCACCCGCAAGGAGGGAGAGTGGCGCTCCCGCGAGGGTGCACTA -501

Sma I

GCCAGATATTCCCTGCGGGCCCGAGAGTCTTCCCTATCAGACCCCGGGATAGGGATGAGGCCCACAGTCACCCACCAGACTCTTTGTATAGCCCCGTTAA -401

GTGCACCCCGGCCTGGAGGGGGTGGTTCTGGGTAGAAGCACGTCCGGGCCGCGCGGATGCCTCCTGGAAGGCGCCTGGACCCACGCCAGGTTTCCCAGTT -301

Sma I

TAATTCCTCATGACTTAGCGTCCCAGCCCGCGCACCGACCAGCGCCCCAGTTCCCCACAGACGCCGGCGGGCCCGGGAGCCTCGCGGACGTGACGCCGCG -201

Sp I Sp I

GGCGGAAGTGACGTTTTCCCGCGGTTGGACGCCGCTCTCAGTTGCCGGGCGGGGGAGGGCGCGTCCGGTTTTTCTCAGGGGACGTTGAAATTATTTTTGT -101

AACGGGAGTCGGGGCAGGACGGGGCGTGCCCCGCGTGCGCGCGCGTCGTCCTCCCCGGCGCTCCTCCACAGCTCGCTGGCTCCCGCCGCGGAAAGGCGTC -1

(#1, >137b) Cfr I, Eag I, Gdl II Dsa I,FnuD II,Sac II Cau II, Hgic I, Hpa II Sau I

ATG CCG CCC AAA ACC CCC CGA AAA ACG GCC GCC ACC GCC GCC GCT GCC GCC GCG GAA CCC CCG GCA CCG CCG CCG CCG CCC CCT CCT GAG 90

M P P K T P R K T A A T A A A A A A E P P A P P P P P P E

Hpa II BspM I 137 __I__ 138 (#2,127b)

GAG GAC CCA GAG CAG GAC AGC GGC CCG GAG GAC CTG CCT CTC GTC AGG CTT GAG TTT GAA GAA ACA GAA GAA CCT GAT TTT ACT GCA TTA 180

E D P E Q D S G P E D L P L V R L E F E E T E E P D F T A L

Hpa I, Hinc II Fok I 264 __I__ 265

TGT CAG AAA TTA AAG ATA CCA GAT CAT GTC AGA GAG AGA GCT TGG TTA ACT TGG GAG AAA GTT TCA TCT GTG GAT GGA GTA TTG GGA GGT 270

C Q K L K I P D H V R E R A W L T W E K V S S V D G V L G G

(#3, 116b) Bbv I Hinc II

TAT ATT CAA AAG AAA AAG GAA CTG TGG GGA ATC TGT ATC TTT ATT GCA GCA GTT GAC CTA GAT GAG ATG TCG TTC ACT TTT ACT GAG CTA 360

Y I Q K K K E L W G I C I F I A A V D L D E M S F T F T E L

380 __I__ 381 (#4,120b)

CAG AAA AAC ATA GAA ATC AGT GTC CAT AAA TTC TTT AAC TTA CTA AAA GAA ATT GAT ACC AGT ACC AAA GTT GAT AAT GCT ATG TCA AGA 450

Q K N I E I S V H K F F N L L K E I D T S T K V D N A M S R

500 __I__ Nsp I, Afl III 501 (#5, 39b) 539 __I__

CTG TTG AAG AAG TAT GAT GTA TTG TTT GCA CTC TTC AGC AAA TTG GAA AGG ACA TGT GAA CTT ATA TAT TTG ACA CAA CCC AGC AGT TCG 540

L L K K Y D V L F A L F S K L E R T C E L I Y L T Q P S S S

Eco RV __ 540 (#6, 68b) 607 __I__ 608 (#7, 111b)

ATA TCT ACT GAA ATA AAT TCT GCA TTG GTG CTA AAA GTT TCT TGG ATC ACA TTT TTA TTA GCT AAA GGG GAA GTA TTA CAA ATG GAA GAT 630

I S T E I N S A L V L K V S W I T F L L A K G E V L Q M E D

```
                                                                                                   718 __|__
GAT CTG GTG ATT TCA TTT CAG TTA ATG CTA TGT GTC CTT GAC TAT TTT ATT AAA CTC TCA CCT CCC ATG TTG CTC AAA GAA CCA TAT AAA    720
 D   L   V   I   S   F   Q   L   M   L   C   V   L   D   Y   F   I   K   L   S   P   P   M   L   L   K   E   P   Y   K
 Pvu II __ 719 (#8, 143b)                                                       ApaL I
ACA GCT GTT ATA CCC ATT AAT GGT TCA CCT CGA ACA CCC AGG CGA GGT CAG AAC AGG AGT GCA CGG ATA GCA AAA CAA CTA GAA AAT GAT    810
 T   A   V   I   P   I   N   G   S   P   R   T   P   R   R   G   Q   N   R   S   A   R   I   A   K   Q   L   E   N   D
                                                     861 __|__ 862 (#9, 78b)
ACA AGA ATT ATT GAA GTT CTC TGT AAA GAA CAT GAA TGT AAT ATA GAT GAG GTG AAA AAT GTT TAT TTC AAA AAT TTT ATA CCT TTT ATG    900
 T   R   I   I   E   V   L   C   K   E   H   E   C   N   I   D   E   V   K   N   V   Y   F   K   N   F   I   P   F   M
Eco RI                                   939 __|__ 940 (#10, 110b)                                                Bgl II
AAT TCT CTT GGA CTT GTA ACA TCT AAT GGA CTT CCA GAG GTT GAA AAT CTT TCT AAA CGA TAC GAA GAA ATT TAT CTT AAA AAT AAA GAT    980
 N   S   L   G   L   V   T   S   N   G   L   P   E   V   E   N   L   S   K   R   Y   E   E   I   Y   L   K   N   K   D
 Xba I                 BspH I                                   1049 __|__ 1050 (#11, 78b)
CTA GAT GCA AGA TTA TTT TTG GAT CAT GAT AAA ACT CTT CAG ACT GAT TCT ATA GAC AGT TTT GAA ACA CAG AGA ACA CCA CGA AAA AGT    1080
 L   D   A   R   L   F   L   D   H   D   K   T   L   Q   T   D   S   I   D   S   F   E   T   Q   R   T   P   R   K   S
                                         Gsu I 1127 __|__ 1128 (#12, 88b)     Mme I
AAC CTT GAT GAA GAG GTG AAT GTA ATT CCT CCA CAC ACT CCA GTT AGG ACT GTT ATG AAC ACT ATC CAA CAA TTA ATG ATG ATT TTA AAT    1170
 N   L   D   E   E   V   N   V   I   P   P   H   T   P   V   R   T   V   M   N   T   I   Q   Q   L   M   M   I   L   N
        Bcl I                                 1215 __|__ 1216 (#13, 117b)             Acc I, Sna I
TCA GCA AGT GAT CAA CCT TCA GAA AAT CTG ATT TCC TAT TTT AAC AAC TGC ACA GTG AAT CCA AAA GAA AGT ATA CTG AAA AGA GTG AAG    1260
 S   A   S   D   H   P   S   E   N   L   I   S   Y   F   N   N   C   T   V   N   P   K   E   S   I   L   K   R   V   K
                                                   Fin I                   1332 __|__ 1333 (#14, 57b)
GAT ATA GGA TAC ATC TTT AAA GAG AAA TTT GCT AAA GCT GTG GGA CAG GGT TGT GTC GAA ATT GGA TCA CAG CGA TAC AAA CTT GGA GTT    1350
 D   I   G   Y   I   F   K   E   K   F   A   K   A   V   G   Q   G   C   V   E   I   G   S   Q   R   Y   K   L   G   V
                                         1389 __|__ 1390 (#15, 32b)           1421 __|__ 1422 (#16, 77b)
CGC TTG TAT TAC CGA GTA ATG GAA TCC ATG CTT AAA TCA GAA GAA GAA CGA TTA TCC ATT CAA AAT TTT AGC AAA CTT CTG AAT GAC AAC    1440
 R   L   Y   Y   R   V   M   E   S   M   L   K   S   E   E   E   R   L   S   I   Q   N   F   S   K   L   L   N   D   N
     Nde I               Hha I, Cfr I              Hae I, Bal I   1498 __|__ 1499 (#17, 197b)
ATT TTT CAT ATG TCT TTA TTG GCG TGC GCT CTT GAG GTT GTA ATG GCC ACA TAT AGC AGA AGT ACA TCT CAG AAT CTT GAT TCT GGA ACA    1530
 I   F   H   M   S   L   L   A   C   A   L   E   V   V   M   A   T   Y   S   R   S   T   S   Q   N   L   D   S   G   T
          Nco I, Dsa I, Sty I
GAT TTG TCT TTC CCA TGG ATT CTG AAT GTG CTT AAT TTA AAA GCC TTT GAT TTT TAC AAA GTG ATC GAA AGT TTT ATC AAA GCA GAA GGC    1620
 D   L   S   F   P   W   I   L   N   V   L   N   L   K   A   F   D   F   Y   K   V   I   E   S   F   I   K   A   E   G
                                                                                    1695 __|__ 1696 (#18, 119b)
AAC TTG ACA AGA GAA ATG ATA AAA CAT TTA GAA CGA TGT GAA CAT CGA ATC ATG GAA TCC CTT GCA TGG CTC TCA GAT TCA CCT TTA TTT    1710
 N   L   T   R   E   M   I   K   H   L   E   R   C   E   H   R   I   M   E   S   L   A   W   L   S   D   S   P   L   F
```

FIG.1-2

FIG. 1-3

Bcl I                                                  Gsu I
GAT CTT ATT AAA CAA TCA AAG GAC CGA GAA GGA CCA ACT GAT CAC CTT GAA TCT GCT TGT CCT CTT AAT CTT CCT CTC CAG AAT AAT CAC 1800
D L I K Q S K D R E G P T D H L E S A C P L N L P L Q N N H

Pst I Bby I 1814 __I__ 1815(#19, 146b) Bgl II Mlu I, FnuD II, Afl III
ACT GCA GCA GAT ATG TAT CTT TCT CCT GTA AGA TCT CCA AAG AAA AAA GGT TCA ACT ACG CGT GTA AAT TCT ACT GCA AAT GCA GAG ACA 1890
T A A D M Y L S P V R S P K K K G S T T R V N S T A N A E T

1960 __I__ 1961 Nhe I (#20,146b)
CAA GCA ACC TCA GCC TTC CAG ACC CAG AAG CCA TTG AAA TCT ACC TCT CTT TCA CTG TTT TAT AAA AAA GTG TAT CGG CTA GCC TAT CTC 1980
Q A T S A F Q T Q K P L K S T S L S L F Y K K V Y R L A Y L

Hpa II Dra III Xmn I Leucine Zipper (L4, 28b) Pst I
CGG CTA AAT ACA CTT TGT GAA CGC CTT CTG TCT GAG CAC CCA GAA TTA GAA CAT ATC ATC TGG ACC CTT TTC CAG CAC ACC CTG CAG AAT 2070
R L N T L C E R L L S E H P E L E H I I W T L F Q H T L Q N

BspH I 2106 __I__ 2107 (#21, 105b) Nde I
GAG TAT GAA CTC ATG AGA GAC AGG CAT TTG GAC CAA ATT ATG ATG TGT TCC ATG TAT GGC ATA TGC AAA GTG AAG AAT ATA GAC CTT AAA 2160
E Y E L M R D R H L D Q I M M C S M Y G I C K V K N I D L K

2211 __I__ 2212 Mae II, Afl III Bcl I (#22, 114b)
TTC AAA ATC ATT GTA ACA GCA TAC AAG GAT CTT CCT CAT GCT GTT CAG GAG ACA TTC AAA CGT GTT TTG ATC AAA GAA GAG GAG TAT GAT 2250
F K I I V T A Y K D L P H A V Q E T F K R V L I K E E E Y D

Ssp I 2325 __I__ 2326(#23,164b)
TCT ATT ATA GTA TTC TAT AAC TCG GTC TTC ATG CAG AGA CTG AAA ACA AAT ATT TTG CAG TAT GCT TCC ACC AGG CCC CCT ACC TTG TCA 2340
S I I V F Y N S V F M Q R L K T N I L Q Y A S T R P P T L S

CCA ATA CCT CAC ATT CCT CGA AGC CCT TAC AAG TTT CCT AGT TCA CCC TTA CGG ATT CCT GGA GGG AAC ATC TAT ATT TCA CCC CTG AAG 2430
P I P H I P R S P Y K F P S S P L R I P G G N I Y I S P L K

2489 __I__ 2490(#24,31b) 2520 Fin I __I
AGT CCA TAT AAA ATT TCA GAA GGT CTG CCA ACA CCA ACA AAA ATG ACT CCA AGA TCA AGA ATC TTA GTA TCA ATT GGT GAA TCA TTC GGG 2520
S P Y K I S E G L P T P T K M T P R S R I L V S I G E S F G

I__ 2521 (#25, 143b)
ACT TCT GAG AAG TTC CAG AAA ATA AAT CAG ATG GTA TGT AAC AGC GAC CGT GTG CTC AAA AGA AGT GCT GAA GGA AGC AAC CCT CCT AAA 2610
T S E K F Q K I N Q M V C N S D R V L K R S A E G S N P P K

2663 __I__ 2664 (#26, 50b)
CCA CTG AAA AAA CTA CGC TTT GAT ATT GAA GGA TCA GAT GAA GCA GAT GGA AGT AAA CAT CTC CCA GGA GAG TCC AAA TTT CAG CAG AAA 2700
P L K K L R F D I E G S D E A D G S K H L P G E S K F Q Q K

2713 __I__ 2714 (#27, 1889b) Bsm I
CTG GCA GAA ATG ACT TCT ACT CGA ACA CGA ATG CAA AAG CAG AAA ATG AAT GAT AGC ATG GAT ACC TCA AAC AAG GAA GAG AAA TGA GGA 2790
L A E M T S T R T R M Q K Q K M N D S M D T S N K E E K *

Sty I | Bal I, Cfr I, Hae I
TCTCAGGACCTTGGTGGACACTGTGTACACCTCTGGATTCATTGTCTCTCACAGATGTGACTGTATAACTTTCCCAGGTTCTGTTTATGGCCACATTTAA 2890

TATCTTCAGCTCTTTTTGTGGATATAAAATGTGCAGATGCAATTGTTTGGGTGATTCCTAAGCCACTTGAAATGTTAGTCATTGTTATTTATACAAGATT 2990

GAAAATCTTGTGTAAATCCTGCCATTTAAAAAGTTGTAGCAGATTGTTTCCTCTTCCAAAGTAAAATTGCTGTGCTTTATGGATAGTAAGAATGGCCCTA 3090

Stu I, Hae I
GAGTGGGAGTCCTGATAACCCAGGCCTGTCTGACTACTTTGCCTTCTTTTGTAGCATATAGGTGATGTTTGCTCTTGTTTTTATTAATTTATATGTATAT 3190

TTTTTTAATTTAACATGAACACCCTTAGAAAATGTGTCCTATCTATCTTCCAAATGCAATTTGATTGACTGCCCATTCACCAAAATTATCCTGAACTCTT 3290

CTGCAAAAATGGATATTATTAGAAATTAGAAAAAAATTACTAATTTTACACATTAGATTTTATTTTACTATTGGAATCTGATATACTGTGTGCTTGTTTT 3390

Nde I
ATAAAATTTTGCTTTTAATTAAATAAAAGCTGGAAGCAAAGTATAACCATATGATACTATCATACTACTGAAACAGATTTCATACCTCAGAATGTAAAAG 3490

Mme I | Sca I
AACTTACTGATTATTTTCTTCATCCAACTTATGTTTTTAAATGAGGATTATTGATAGTACTCTTGGTTTTTATACCATTCAGATCACTGAATTTATAAAG 3590

Sca I | Bgl II | ApaL I
TACCCATCTAGTACTTGAAAAAGTAAAGTGTTCTGCCAGATCTTAGGTATAGAGGACCCTAACACAGTATATCCCAAGTGCACTTTCTAATGTTTCTGGG 3690

TCCTGAAGAATTAAGATACAAATTAATTTTACTCCATAAACAGACTGTTAATTATAGGAGCCTTAATTTTTTTTTCATAGAGATTTGTCTAATTGCATCT 3790

Nsp I, Afl III
CAAAATTATTCTGCCCTCCTTAATTTGGGAAGGTTTGTGTTTTCTCTGGAATGGTACATGTCTTCCATCTATCTTTTGAACTGGCAATTGTCTATTTATC 3890

Nsp I
TTTTATTTTTTTAAGTCAGTATGGTCTAACACTGGCATGTTCAAAGCCACATTATTTCTAGTCCAAAATTACAAGTAATCAAGGGTCATTATGGGTTAGG 3990

Hind III | Pvu II | Hha I, Hae II
CATTAATGTTTCTATCTGATTTTGTGCAAAAGCTTCAAATTAAAACAGCTGCATTAGAAAAAGAGGCGCTTCTCCCCTCCCCTACACCTAAAGGTGTATT 4090

Nhe I
TAAACTATCTTGTGTGATTAACTTATTTAGAGATGCTGTAACTTAAAATAGGGGATATTTAAGGTAGCTTCAGCTAGCTTTTAGGAAAATCAGTTTGTCT 4190

AACTCAGAATTATTTTTAAAAAGAAATCTGGTCTTGTTAGAAAAGAAAATTTTATTTTGTGCTCATTTAAGTTTCAAACTTACTATTTTGACAGTTATTT 4290

TGATAACAATGACACTAGAAAACTTGACTCCATTTCATCATTGTTTCTGCATGAATATCATACAAATCAGTTAGTTTTTAGGTCAAGGGCTTACTATTTC 4390

TGGGTCTTTTGCTACTAAGTTCACATTAGAATTAGTGCCAGAATTTTAGGAACTTCAGAGATCGTGTATTGAGATTTCTTAAATAATGCTTCAGATATTA 4490

TTGCTTTATTGCTTTTTTGTATTGGTTAAAACTGTACATTTAAAATTGCTATGTTACTATTTTCTACAATTAATAGTTTGTCTATTTTAAAATAAATTAG 4590

4692
TTGTTAAGAGTCAAAAAAAACCCGAATTC 3'

FIG. 1-4

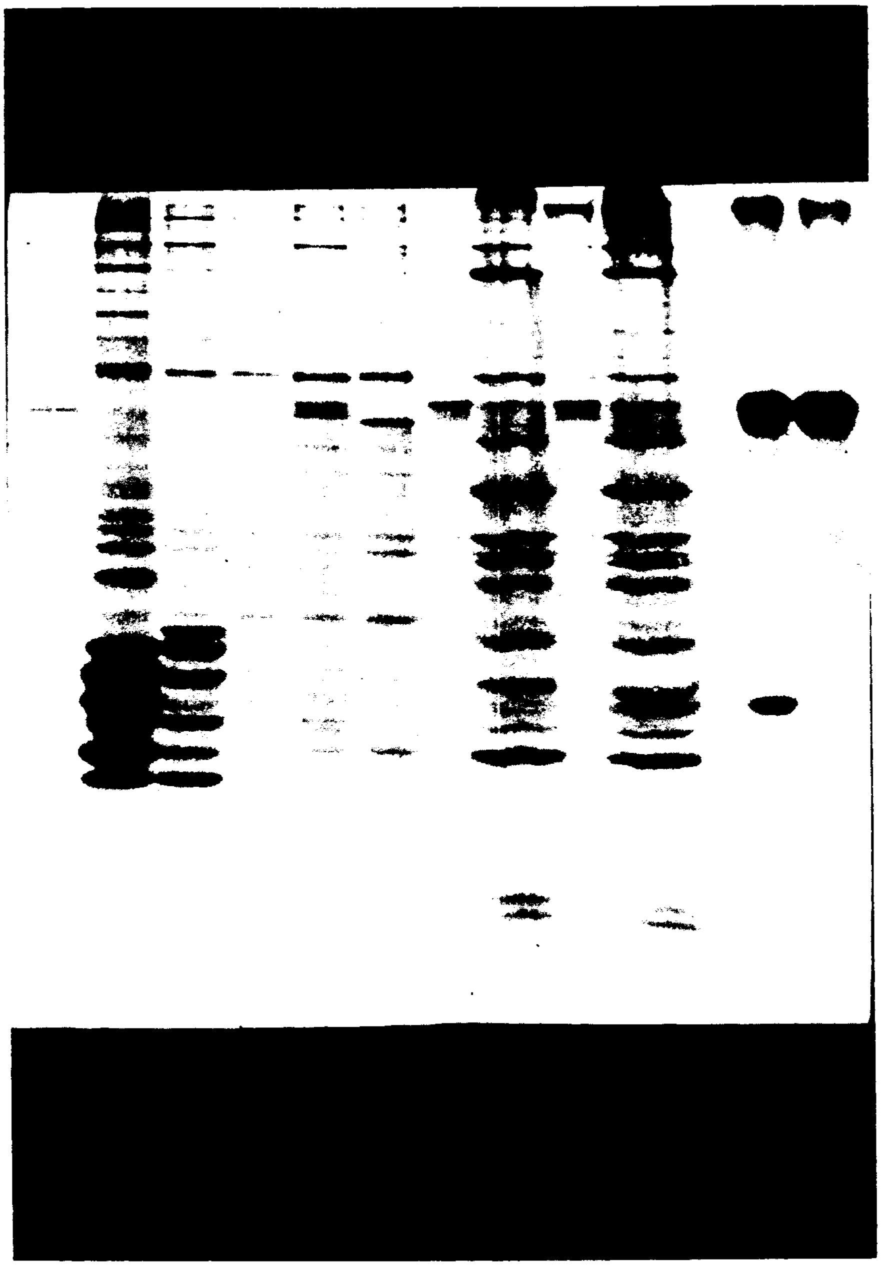

FIG.2

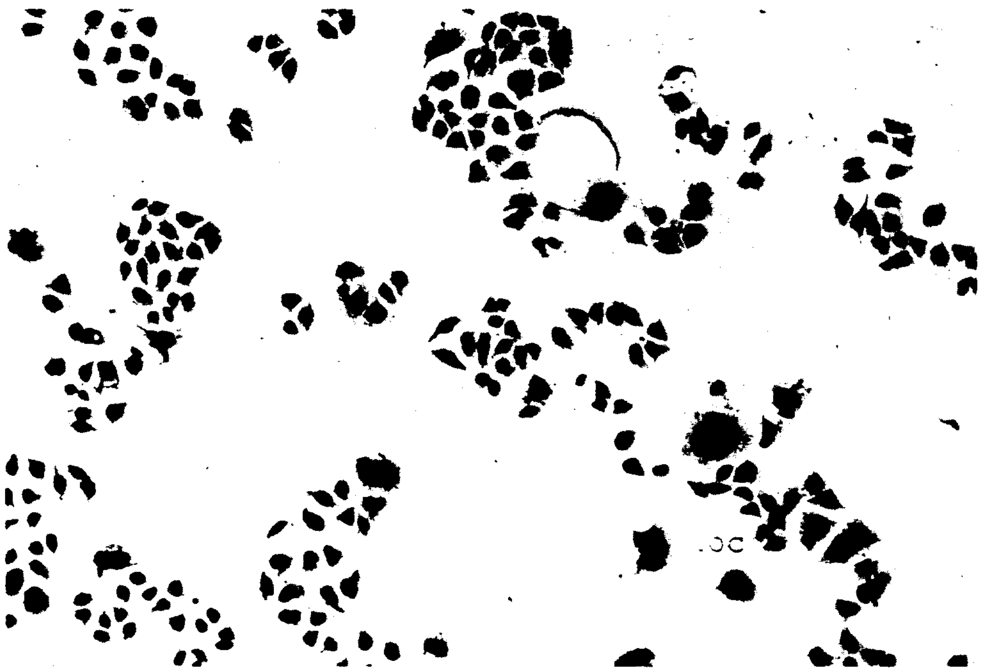

FIG.3a

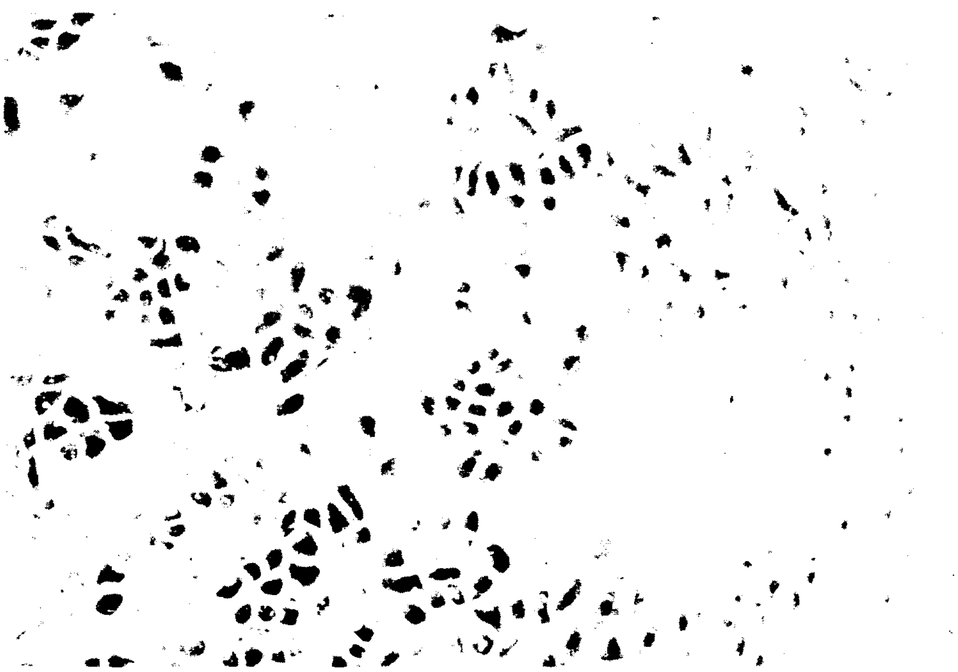

FIG.3b

FIG.4a

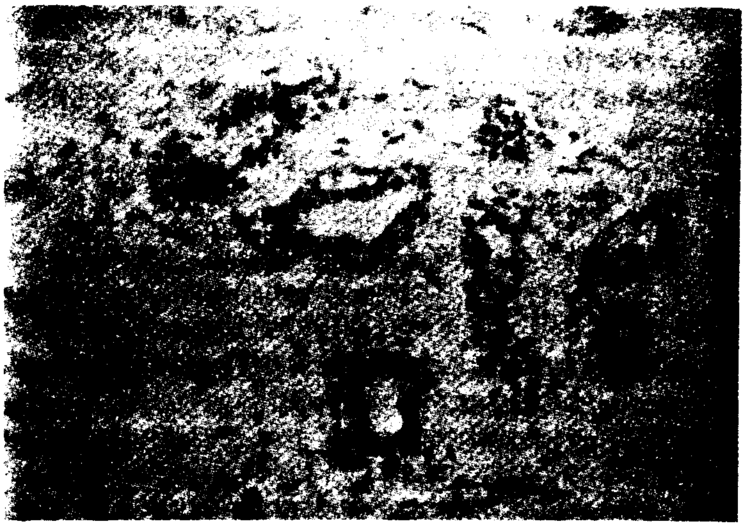

FIG.4b

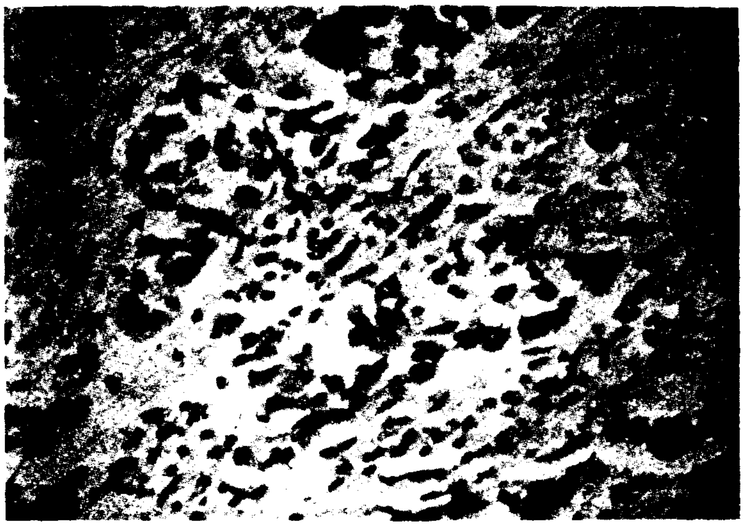

FIG.4c